# EUROPEAN PATENT SPECIFICATION

(11) **EP 2 764 024 B1**
(45) Date of publication and mention of the grant of the patent: **28.08.2019**
(21) Application number: 12838709.9
(22) Date of filing: 05.10.2012
(51) Int. Cl.: C07K 16/28, A61K 39/395, A61P 35/00

(54) **ANTI C-MET ANTIBODY AND USES THEREOF**
ANTI-C-MET-ANTIKÖRPER UND VERWENDUNGEN DAVON
ANTICORPS ANTI C-MET ET UTILISATION DUDIT ANTICORPS

(30) Priority: 05.10.2011 KR 20110101293; 09.07.2012 KR 20120074691
(43) Date of publication of application: 13.08.2014
(73) Proprietor: Samsung Electronics Co., Ltd., Suwon, Gyeonggi-do 443-742 (KR)
(72) Inventor: LEE, Seung-Hyun, Suwon-si Gyeonggi-do 443-400 (KR); KIM, Geu Woong, Yongin-si Gyonggi-do (KR); KIM, Kyung-Ah, Seongnam-si Gyeonggi-do (KR); PARK, Hye-Won, Pyeongtaek-si Gyeonggi-do 420-150 (KR); SONG, Ho-Yeong, Seongnam-si Gyeonggi-do (KR); OH, Young-Mi, Incheon (KR); LEE, Saet-Byoul, Seoul 121-110 (KR); LEE, Ji-Min, Seoul 153-130 (KR); CHEONG, Kwang-Ho, Seoul (KR); JEONG, Yun-Ju, Geonggi-do (KR); CHO, Mi-Young, Seoul (KR); CHOI, Jae-Hyun, Seongnam-si Gyeonggi-do 463-400 (KR); SONG, Yun Jeong, Seongnam-si Gyeonggi-do 463-410 (KR); CHOI, Yoon-AA, Busan 613-010 (KR)
(74) Representative: Grünecker Patent- und Rechtsanwälte PartG mbB
(86) International application number: PCT/KR2012/008089
(87) International publication number: WO 2013/051891

(56) References cited:
- EP-A1- 2 316 484
- EP-A1- 2 708 556
- WO-A1-2005/058965
- WO-A1-2010/059654
- WO-A2-2006/015371
- WO-A2-2011/110642
- WO-A2-2013/051878
- CN-A- 102 174 106
- US-A1- 2005 054 019
- US-A1- 2010 115 639
- US-A1- 2010 129 369
- US-A1- 2010 254 989

## Description

### Technical Field

The present invention relates to an anti c-Met antibody and a pharmaceutical composition comprising same for use in preventing or treating cancer.

### Description of the Related Art

c-Met is a receptor for hepatocyte growth factor (HGF), a cytokine that binds the extracellular region of the c-Met receptor tyrosine kinase to induce cell division, movement, morphogenesis, and angiogenesis of various normal cells and tumor cells. c-Met is a representative receptor tyrosine kinase existing on the surface of cells, is itself a proto-oncogene, and is sometimes involved in various mechanisms related to cancer, such as cancer development, metastasis, migration, invasion, and angiogenesis, independent from its ligand, HGF. Thus, c-Met has been recently emerging as a new target for anti-cancer therapy.

In particular, c-Met is known to be involved in induction of resistance to commonly used anti-cancer drugs, and thus is regarded as important with respect to personalized treatments. Representative anti-cancer therapeutic drugs targeting epidermal growth factor receptor EGFR (ERBB1), i.e., Eribitux or Tarceva, work by blocking the signaling related to cancer development. In addition, Herceptin, which is well known as a breast cancer therapeutic drug, targets ERBB2 (HER2) and works by blocking the transduction of signals necessary for cell proliferation. Among patients resistant to the drugs described above, the signal transduction pathway that induces cell proliferation is not blocked due to the overexpression of c-Met. Thus, c-Met has emerged as a target of interest for many pharmaceutical companies. Still, there is a need for additional anti-c-Met antibodies and related methods and compositions. EP 2 316 484 A1 discloses a monoclonal antibody called AbF46 produced from mouse hybridoma cells that binds to the extracellular region of the c-Met antigen.

### SUMMARY

Provided is an anti c-Met antibody or an antigen-binding fragment thereof, as defined in the appended claims. In particular, the invention provides an anti c-Met antibody or antigen-binding fragment thereof comprising: a heavy chain variable region comprising the following heavy chain complementarity determining regions (CDRs): a) CDR-H1 having an amino acid sequence of SEQ ID NO: 1, b) CDR-H2 having an amino acid sequence of SEQ ID NO: 2, and c) CDR-H3 having an amino acid sequence of SEQ ID NO: 3; and a light chain variable region comprising the following light chain CDRs: a) CDR-L1 having an amino acid sequence of SEQ ID NO: 10 or 71, b) CDR-L2 having an amino acid sequence of SEQ ID NO: 11, and c) CDR-L3 having an amino acid sequence of SEQ ID NO: 13, 14, 15, or 16. In one disclosure, the anti c-Met antibody or antigen-binding fragment thereof comprises a heavy chain variable region comprising at least one heavy chain complementarity determining region (CDR) selected from the group consisting of CDR-H1 having an amino acid sequence of SEQ ID NO: 4, CDR-H2 having an amino acid sequence of SEQ ID NO: 5, and CDR-H3 having an amino acid sequence of SEQ ID NO: 6; and a light chain variable region comprising at least one light chain CDR selected from the group consisting of CDR-L1 having an amino acid sequence of SEQ ID NO: 7, CDR-L2 having an amino acid sequence of SEQ ID NO: 8, and CDR-L3 having an amino acid sequence of SEQ ID NO: 9, wherein SEQ ID NOs: 4 to 9 are respectively represented by Formulas I to VI, described herein. In another disclosure, the anti c-Met antibody or antigen-binding fragment thereof comprises a heavy chain variable region comprising at least one heavy chain complementarity determining region (CDR) selected from the group consisting of CDR-H1 having an amino acid sequence of SEQ ID NO: 1, CDR-H2 having an amino acid sequence of SEQ ID NO: 2, and CDR-H3 having an amino acid sequence of SEQ ID NO: 3; and a light chain variable region comprising at least one light chain CDR selected from the group consisting of CDR-L1 having an amino acid sequence of SEQ ID NO: 7, CDR-L2 having an amino acid sequence of SEQ ID NO: 8, and CDR-L3 having an amino acid sequence of SEQ ID NO: 9, wherein SEQ ID NOS: 7 to 9 are respectively represented by Formulas IV to VI described herein. Nucleic acids encoding the antibodies and antibody fragments also are provided.

Further provided is a pharmaceutical composition including an anti c-Met antibody or an antigen-binding fragment thereof, as well as the antibody or antigen-binding fragment thereof for use in preventing or treating cancer.

### BRIEF DESCRIPTION OF THE DRAWINGS

These and/or other aspects will become apparent and more readily appreciated from the following description of the embodiments, taken in conjunction with the accompanying drawings of which:
FIG. 1 is a diagram showing the use of overlap extension PCR to obtain a scFv gene library of huAbF46 antibodies in which a desired CDR is mutated;
FIG. 2 is a graph of BrdU (%) plotted against antibody concentration, showing c-Met agonistic effect of huAbF46-H4-A1, huAbF46-H4-A2, huAbF46-H4-A3, and huAbF46-H4-A5 antibodies in a BrdU assay;
FIG. 3 is a graph of relative cell viability (%) plotted against antibody concentration, illustrating of the effect of huAbF46-H4-A1, huAbF46-H4-A2, huAbF46-H4-A3, and huAbF46-H4-A5 antibodies on in vitro cell proliferation;
FIG. 4 is a graph of Akt phosphorylation (%) plotted against treatment antibody, which shows the degree of agonism of huAbF46-H4-A1, huAbF46-H4-A2, huAbF46-H4-A3, and huAbF46-H4-A5 antibodies;
FIG. 5 is a graph illustrating anti-cancer effects of huAbF46-H4-A1, huAbF46-H4-A2, huAbF46-H4-A3, and huAbF46-H4-A5 antibodies as measured by the degree of degradation of c-Met;
FIGS. 6A and 6B are graphs of tumor volume plotted against time (days), showing in vivo anti-cancer effects of various concentrations of huAbF46-H4-A1 antibodies in a U87MG brain cancer mouse xenograft model or MKN45 gastric cancer mouse xenograft model;
FIG. 7 is a graph of relative cell viability (%) plotted against antibody concentration, showing the effect of huAbF46-H4-A1 (U6-HC7), huAbF46-H4-A1 (IgG2 hinge), and huAbF46-H4-A1 (IgG2 Fc) antibodies on in vitro cell proliferation;
FIGS. 8A and 8B are graphs of Akt phosphorylation (%) plotted against treatment antibody, which shows the degree of agonism of the antibodies. FIG. 8A shows the degree of Akt phosphorylation by huAbF46-H4-A1 (U6-HC7), huAbF46-H4-A1 (IgG2 hinge), and huAbF46-H4-A1 (IgG2 Fc) antibodies, and FIG. 8B shows the degree of Akt phosphorylation by huAbF46-H4-A1 (IgG2 Fc) and L3-11Y antibodies;
FIGS. 9A and 9B are graphs illustrating anti-cancer effects of antibodies as measured by degree of degradation of c-Met. FIG. 9A shows the degree of degradation of c-Met by huAbF46-H4-A1 (U6-HC7), huAbF46-H4-A1 (IgG2 hinge), and huAbF46-H4-A1 (IgG2 Fc) antibodies, and FIG. 9B shows the degree of degradation of c-Met by huAbF46-H4-A1 (IgG2 Fc) and L3-11Y antibodies; and
FIGS. 10A and 10B are graphs of tumor volume plotted against time (days), showing in vivo anti-cancer effects of huAbF46-H4-A1 (U6-HC7), huAbF46-H4-A1 (IgG2 hinge), and huAbF46-H4-A1 (IgG2 Fc) antibodies in a U87MG brain cancer mouse xenograft model or MKN45 gastric cancer mouse xenograft model.

### DETAILED DESCRIPTION

The invention is defined in the appended claims. Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one" when preceding a list of elements, modify the entire list of elements and do not modify the individual elements of the list.

The invention provides an anti c-Met antibody or antigen-binding fragment thereof comprising: a heavy chain variable region comprising the following heavy chain complementarity determining regions (CDRs): a) CDR-H1 having an amino acid sequence of SEQ ID NO: 1, b) CDR-H2 having an amino acid sequence of SEQ ID NO: 2, and c) CDR-H3 having an amino acid sequence of SEQ ID NO: 3; and a light chain variable region comprising the following light chain CDRs: a) CDR-L1 having an amino acid sequence of SEQ ID NO: 10 or 71, b) CDR-L2 having an amino acid sequence of SEQ ID NO: 11, and c) CDR-L3 having an amino acid sequence of SEQ ID NO: 13, 14, 15, or 16. According to a disclosure, there is disclosed an anti c-Met antibody or an antigen-binding fragment thereof, wherein the antibody includes: a heavy chain variable region having the amino acid sequence of at least one heavy chain complementarity determining region (CDR) selected from the group consisting of CDR-H1 having an amino acid sequence of SEQ ID NO: 4, CDR-H2 having an amino acid sequence of SEQ ID NO: 5, and CDR-H3 having an amino acid sequence of SEQ ID NO: 6; and a light chain variable region having the amino acid sequence of at least one light chain complementarity determining region selected from the group consisting of CDR-L1 having an amino acid sequence of SEQ ID NO: 7, CDR-L2 having an amino acid sequence of SEQ ID NO: 8, and CDR-L3 having an amino acid sequence of SEQ ID NO: 9, in which SEQ ID NOS: 4 to 9 are respectively represented by Formula I to VI below:
Formula I
   Xaa₁-Xaa₂-Tyr-Tyr-Met-Ser (SEQ ID NO: 4)
Formula II
   Arg-Asn-Xaa₃-Xaa₄-Asn-Gly-Xaa₅-Thr (SEQ ID NO: 5)
Formula III
   Asp-Asn-Trp-Leu-Xaa₆-Tyr (SEQ ID NO: 6)
Formula IV
   Lys-Ser-Ser-Xaa₇-Ser-Leu-Leu-Ala-Xaaₐ-Gly-Asn-Xaa₉-Xaa₁₀-Asn-Tyr-Leu-Ala (SEQ ID NO: 7)
Formula V
   Trp-Xaa₁₁-Ser-Xaa₁₂-Arg-Val-Xaa₁₃ (SEQ ID NO: 8)
Formula VI
   Xaa₁₄-Gln-Ser-Tyr-Ser-Xaa₁₅-Pro-Xaa₁₆-Thr (SEQ ID NO: 9)

In Formula I, Xaa₁ is Pro or Ser or absent, and Xaa₂ is Glu or Asp.

In Formula II, Xaa₃ is Asn or Lys, Xaa₄ is Ala or Val, and Xaa₅ is Asn or Thr.

In Formula III, Xaa₆ is Ser or Thr.

In Formula IV, Xaa₇ is His, Arg, Gln, or Lys, Xaa₈ is Ser or Trp, Xaag is His or Gln, and Xaa₁₀ is Lys or Asn.

In Formula V, Xaa₁₁ is Ala or Gly, Xaa₁₂ is Thr or Lys, and Xaa₁₃ is Ser or Pro.

In Formula VI, Xaa₁₄ is Gly, Ala, or Gln, Xaa₁₅ is Arg, His, Ser, Ala, Gly, or Lys, and Xaa₁₆ is Leu, Tyr, Phe, or Met.

For example, the CDR-H1 is disclosed to be a polypeptide having one amino acid sequence selected from the group consisting of amino acid sequences of SEQ ID NOS: 22 to 24, the CDR-H2 is disclosed to be a polypeptide having an amino acid sequence of SEQ ID NO: 25 or 26, and the CDR-H3 is disclosed to be a polypeptide having an amino acid sequence of SEQ ID NO: 27 or 28.

Also, the CDR-L1 is disclosed to be a polypeptide having one amino acid sequence selected from the group consisting of amino acid sequences of SEQ ID NOS: 29 to 33 and 71, CDR-L2 is disclosed to be a polypeptide having one amino acid sequence selected from the group consisting of amino acid sequences of SEQ ID NOS: 34 to 36, and CDR-L3 is disclosed to be a polypeptide having one amino acid sequence selected from the group consisting of amino acid sequences of SEQ ID NOS: 13 to 16 or a polypeptide having an amino acid sequence of SEQ ID NO: 37.

According to another disclosure, there is disclosed an anti c-Met antibody or antigen binding fragment thereof including: a heavy chain variable region having an amino acid sequence of at least one heavy chain complementarity determining region selected from the group consisting of CDR-H1 having an amino acid sequence of SEQ ID NO: 1, CDR-H2 having an amino acid sequence of SEQ ID NO: 2, and CDR-H3 having an amino acid sequence of SEQ ID NO: 3; and a light chain variable region having an amino acid sequence of at least one light chain complementarity determining region selected from the group consisting of CDR-L1 having an amino acid sequence of SEQ ID NO: 7, CDR-L2 having an amino acid sequence of SEQ ID NO: 8, and CDR-L3 having an amino acid sequence of SEQ ID NO: 9, wherein SEQ ID NOS: 7 to 9 are respectively represented by Formula IV to VI below:
Formula IV
   Lys-Ser-Ser-Xaa₇-Ser-Leu-Leu-Ala-Xaa₈-Gly-Asn-Xaa₉-Xaa₁₀-Asn-Tyr-Leu-Ala (SEQ ID NO: 7)
Formula V
   Trp-Xaa₁₁-Ser-Xaa₁₂-Arg-Val-Xaa₁₃ (SEQ ID NO: 8)
Formula VI
   Xaa₁₄-Gln-Ser-Tyr-Ser-Xaa₁₅-Pro-Xaa₁₆-Thr (SEQ ID NO: 9)

In Formula IV, Xaa₇ is His, Arg, Gln, or Lys, Xaa₈ is Ser or Trp, Xaa₉ is His or Gln, and Xaa₁₀ is Lys or Asn.

In Formula V, Xaa₁₁ is Ala or Gly, Xaa₁₂ is Thr or Lys, and Xaa₁₃ is Ser or Pro.

In Formula VI, Xaa₁₄ is Gly, Ala, or Gln, Xaa₁₅ is Arg, His, Ser, Ala, Gly, or Lys, and Xaa₁₆ is Leu, Tyr, Phe, or Met.

According to the invention, the light chain variable region has an amino acid sequence of at least one light chain complementarity determining region selected from the group consisting of CDR-L1 having an amino acid sequence of SEQ ID NO: 10 or 71, CDR-L2 having an amino acid sequence of SEQ ID NO: 11, and CDR-L3 having one amino acid sequence selected from the group consisting of amino acid sequences of SEQ ID NOS: 13 to 16.

By way of further illustration, the heavy chain variable region may have an amino acid sequence of SEQ ID NO: 17, and the light chain variable region may have one amino acid sequence selected from the group consisting of amino acid sequences of SEQ ID NOS: 18 to 21 and 72.

The terms "c-Met" or "c-Met protein" may refer to a receptor tyrosine kinase (RTK) that binds to a hepatocyte growth factor (HGF). c-Met can be a c-Met protein from any species, particularly a mammal or primate, for instance, human c-Met (e.g., NP_000236), or monkey c-Met (e.g., Macaca mulatta, NP_001162100), or rodents such as mouse c-Met (e.g., NP_032617.2), rat c-Met (e.g., NP_113705.1), and the like. The c-Met protein may include a polypeptide encoded by the nucleotide sequence identified as GenBank Accession Number NM_000245, a polypeptide having the amino acid sequence identified as GenBank Accession Number NP_000236 or extracellular domains thereof. The receptor tyrosine kinase c-Met participates in various mechanisms, such as cancer development, metastasis, migration of cancer cell, invasion of cancer cell, and angiogenesis.

Animal-derived antibodies produced by immunizing non-immune animals with a desired antigen generally invoke immunogenicity when injected to humans for the purpose of medical treatment, and thus chimeric antibodies have been developed to inhibit such immunogenicity. Chimeric antibodies are prepared by replacing constant regions of animal-derived antibodies that cause an anti-isotype response with constant regions of human antibodies by genetic engineering. Chimeric antibodies are considerably improved in an anti-isotype response compared to animal-derived antibodies, but animal-derived amino acids still have variable regions, so that chimeric antibodies have side effects with respect to a potential anti-idiotype response. Humanized antibodies are developed to reduce such side effects. Humanized antibodies are produced by grafting complementarity determining regions (CDR) which serve an important role in antigen binding in variable regions of chimeric antibodies into a human antibody framework.

The most important thing in CDR grafting to produce humanized antibodies is choosing the optimized human antibodies for accepting CDR of animal-derived antibodies. Antibody database, analysis of a crystal structure, and technology for molecule modeling are used. However, even when the CDRs of animal-derived antibodies are grafted to the most optimized human antibody framework, amino acids positioned in a framework of the animal-derived CDRs affecting antigen binding are present. Therefore, in many cases, antigen binding affinity is not maintained, and thus application of additional antibody engineering technology for recovering the antigen binding affinity is necessary.

The anti c-Met antibodies may be mouse-derived antibodies, mouse-human chimeric antibodies, or humanized antibodies. The antibodies or antigen-binding fragments thereof may be one previously isolated from a living body.

The antibody may be a monoclonal antibody.

An intact antibody includes two full-length light chains and two full-length heavy chains, in which each light chain is linked to a heavy chain by disulfide bonds. The antibody has a heavy chain constant region and a light chain constant region. The heavy chain constant region is of a gamma (γ), mu (µ), alpha (α), delta (δ), or epsilon (ε) type, which may be further categorized as gamma 1 (γ1), gamma 2(γ2), gamma 3(γ3), gamma 4(γ4), alpha 1(α1), or alpha 2(α2). The light chain constant region is of either a kappa (κ) or lambda (λ) type.

The term "heavy chain" refers to full-length heavy chain, and fragments thereof, including a variable region V_{H} that includes amino acid sequences sufficient to provide specificity to antigens, and three constant regions, C_{H1}, C_{H2}, and C_{H3}, and a hinge. The term "light chain" refers to a full-length light chain and fragments thereof, including a variable region V_{L} that includes amino acid sequences sufficient to provide specificity to antigens, and a constant region C_{L}.

The term "complementarity determining region (CDR)" refers to an amino acid sequence found in a hyper variable region of a heavy chain or a light chain of immunoglobulin. The heavy and light chains may respectively include three CDRs (CDRH1, CDRH2, and CDRH3; and CDRL1, CDRL2, and CDRL3). The CDR may provide contact residues that play an important role in the binding of antibodies to antigens or epitopes. The terms "specifically binding" or "specifically recognized" is well known to one of ordinary skill in the art, and indicates that an antibody and an antigen specifically interact with each other to lead to an immunological activity.

According to an embodiment, the antibody may be an antigen-binding fragment selected from the group consisting of scFv, (scFv)₂, Fab, Fab', and F(ab')₂.

The term "antigen-binding fragment" used herein refers to fragments of an intact immunoglobulin including portions of a polypeptide including antigen-binding regions having the ability to specifically bind to the antigen. For example, the antigen-binding fragment may be scFv, (scFv)₂, Fab, Fab', or F(ab')₂, but is not limited thereto. Among the antigen-binding fragments, Fab that includes light chain and heavy chain variable regions, a light chain constant region, and a first heavy chain constant region C_{H1}, has one antigen-binding site. The Fab' fragment is different from the Fab fragment, in that Fab' includes a hinge region with at least one cysteine residue at the C-terminal of C_{H1}. The F(ab')₂ antibody is formed through disulfide bridging of the cysteine residues in the hinge region of the Fab' fragment. Fv is the smallest antibody fragment with only a heavy chain variable region and a light chain variable region. Recombination techniques of generating the Fv fragment are widely known in the art. Two-chain Fv includes a heavy chain variable region and a light chain region which are linked by a non-covalent bond. Single-chain Fv generally includes a heavy chain variable region and a light chain variable region which are linked by a covalent bond via a peptide linker or linked at the C-terminals to have a dimer structure like the two-chain Fv. The antigen-binding fragments may be attainable using protease (for example, the Fab fragment may be obtained by restricted cleavage of a whole antibody with papain, and the F(ab')₂ fragment may be obtained by cleavage with pepsin), or may be prepared by using a genetic recombination technique.

By way of further example, the anti c-Met antibody or antibody fragment may include a heavy chain with the amino acid sequence of SEQ ID NO: 62 (wherein the amino acid sequence from 1^{st} to 17^{th} is a signal peptide) or the amino acid sequence from 18^{th} to 462^{nd} of SEQ ID NO: 62 and a light chain with the amino acid sequence of SEQ ID NO: 68 (wherein the amino acid sequence from 1^{st} to 20^{th} is a signal peptide) or the amino acid sequence from 21^{st} to 240^{th} of SEQ ID NO: 68; or a heavy chain with the amino acid sequence of SEQ ID NO: 64 (wherein the amino acid sequence from 1^{st} to 17^{th} is a signal peptide) or the amino acid sequence from 18^{th} to 461^{st} of SEQ ID NO: 64 and a light chain with the amino acid sequence of SEQ ID NO: 68 or the amino acid sequence from 21^{st} to 240^{th} of SEQ ID NO: 68; or a heavy chain with the amino acid sequence of SEQ ID NO: 66 (wherein the amino acid sequence from 1^{st} to 17^{th} is a signal peptide) or the amino acid sequence from 18^{th} to 460^{th} of SEQ ID NO: 66 and a light chain with the amino acid sequence of SEQ ID NO: 68 or the amino acid sequence from 21^{st} to 240^{th} of SEQ ID NO: 68.

Additional examples of anti c-Met antibodies include those in which the anti c-Met antibody includes a heavy chain with the amino acid sequence of SEQ ID NO: 62 or the amino acid sequence from 18^{th} to 462^{nd} of SEQ ID NO: 62 and a light chain with the amino acid sequence of SEQ ID NO: 70 (wherein the amino acid sequence from 1^{st} to 20^{th} is a signal peptide) or the amino acid sequence from 21^{st} to 240^{th} of SEQ ID NO: 70; a heavy chain with the amino acid sequence of SEQ ID NO: 64 or the amino acid sequence from 18^{th} to 461^{st} of SEQ ID NO: 64 and a light chain with the amino acid sequence of SEQ ID NO: 70 or the amino acid sequence from 21^{st} to 240^{th} of SEQ ID NO: 70; or a heavy chain with the amino acid sequence of SEQ ID NO: 66 or the amino acid sequence from 18^{th} to 460^{th} of SEQ ID NO: 66 and a light chain with the amino acid sequence of SEQ ID NO: 70 or the amino acid sequence from 21^{st} to 240^{th} of SEQ ID NO: 70.

In still other examples, the anti c-Met antibody may include a heavy chain with the amino acid sequence of SEQ ID NO: 62 or the amino acid sequence from 18^{th} to 462^{nd} of SEQ ID NO: 62 and a light chain with the amino acid sequence of SEQ ID NO: 73; a heavy chain with the amino acid sequence of SEQ ID NO: 64 or the amino acid sequence from 18^{th} to 461^{st} of SEQ ID NO: 64 and a light chain with the amino acid sequence of SEQ ID NO: 73; or a heavy chain with the amino acid sequence of SEQ ID NO: 66 or the amino acid sequence from 18^{th} to 460^{th} of SEQ ID NO: 66 and a light chain with the amino acid sequence of SEQ ID NO: 73.

In an embodiment, the anti c-Met antibody may include a heavy chain with the amino acid sequence from 18^{th} to 460^{th} of SEQ ID NO: 66 and a light chain with the amino acid sequence from 21^{st} to 240^{th} of SEQ ID NO: 68; or a heavy chain with the amino acid sequence from 18^{th} to 460^{th} of SEQ ID NO: 66 and a light chain with the amino acid sequence of SEQ ID NO: 73.

The signal peptides used in the above amino acid sequences are only for exemplifying, and amino acid sequence of signal peptide may be properly determined or modified by a person skilled in the art.

Also disclosed herein is a polypeptide comprising the amino acid sequence of SEQ ID NO: 68 or 73. The polypeptide with the amino acid sequence of SEQ ID NO: 70 is a light chain including human kappa (κ) constant region, and the polypeptide with the amino acid sequence of SEQ ID NO: 68 is a polypeptide obtained by replacing histidine at position 62 (position 36 according to kabat numbering) of the polypeptide with the amino acid sequence of SEQ ID NO: 70 with tyrosine. The production yield of the antibodies may be increased by the replacement. The polypeptide with the amino acid sequence of SEQ ID NO: 73 is a polypeptide obtained by replacing serine at position 32 (position 27e according to kabat numbering; positioned within CDR-L1) of the polypeptide with the amino acid sequence from 21^{st} to 240^{th} positions of SEQ ID NO: 68 with tryptophan. By such replacement, antibodies and antibody fragments comprising such sequences exhibits increased activities, such as c-Met biding affinity, c-Met degradation activity, Akt phosphorylation activity, and the like.

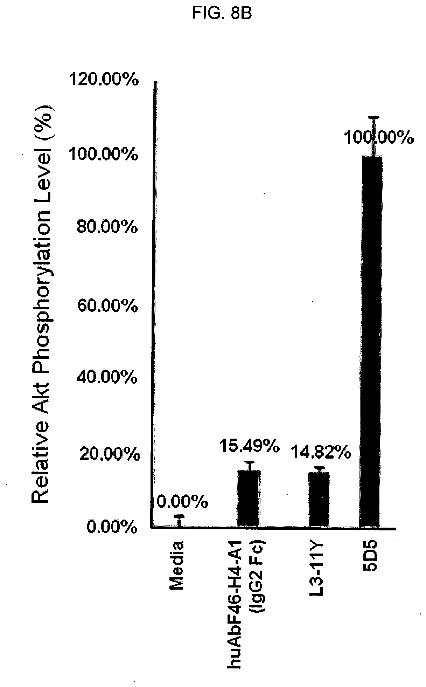

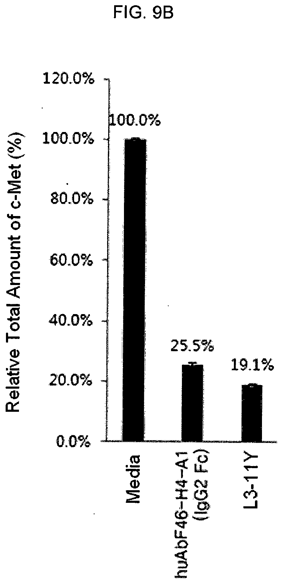

Also disclosed is a polypeptide having the amino acid sequence of SEQ ID NO: 71, which is useful as a light chain complementarity determining region (CDR-L1). Further disclosed is an anti c-Met antibody or an antigen-binding fragment thereof including a light chain complementarity determining region having the amino acid sequence of SEQ ID NO: 71, a light chain variable region having the amino acid sequence of SEQ ID NO: 72, or a light chain having the amino acid sequence of SEQ ID NO: 73, optionally in combination with a heavy chain variable region or heavy chain as described herein, or other heavy chain that provides an anti c-Met antibody or antibody fragment. The antibody or the antigen-binding fragment thereof exhibits increased c-Met degradation activity and Akt phosphorylation activity, as shown in FIGS. 8B and 9B.

According to another embodiment, there is provided a pharmaceutical composition including the anti c-Met antibody or the antigen-binding fragment as an active ingredient. The pharmaceutical composition can be used for preventing or treating cancer, and may include a pharmaceutically effective amount of the anti c-Met antibody or the antigen-binding fragment; and a pharmaceutically acceptable carrier, a diluent, or an excipient. The cancer may be any cancer associated with c-Met activity. The cancer may be any selected from the group consisting of squamous cell carcinoma, small-cell lung cancer, non-small-cell lung cancer, adenocarcinoma of the lung, squamous cell carcinoma of the lung, peritoneal carcinoma, skin cancer, skin or intraocular melanoma, colorectal cancer, cancer near the anus, esophagus cancer, small intestinal tumor, endocrine gland cancer, parathyroid cancer, adrenal cancer, soft-tissue sarcoma, urethral cancer, chronic or acute leukemia, lymphocytic lymphoma, hepatoma, gastrointestinal cancer, pancreatic cancer, glioblastoma, cervical cancer, ovarian cancer, liver cancer, bladder cancer, hepatocellular adenoma, breast cancer, colon cancer, large intestine cancer, endometrial or uterine carcinoma, salivary gland tumor, kidney cancer, prostate cancer, vulvar cancer, thyroid cancer, and head and neck cancers.

The pharmaceutical composition may include a pharmaceutically acceptable carrier, a diluent, and/or excipient. The pharmaceutically acceptable carriers included in the composition may include commonly used lactose, dextrose, sucrose, sorbitol, mannitol, starch, gum acacia, calcium phosphate, alginates, gelatin, calcium silicate, microcrystalline cellulose, polyvinylpyrrolidone, cellulose, water, syrup, methyl cellulose, methylhydroxy benzoate, propylhydroxy benzoate, talc, magnesium stearate, and mineral oil, but is not limited thereto. The pharmaceutical composition may further include a lubricant, a wetting agent, a sweetener, a flavor enhancer, an emulsifying agent, a suspension agent, and a preservative.

The pharmaceutical composition may be administered orally or parenterally. The parenteral administration may include intravenous injection, subcutaneous injection, muscular injection, intraperitoneal injection, endothelial administration, local administration, intranasal administration, intrapulmonary administration, and rectal administration. Since oral administration leads to digestions of protein or peptide, an active ingredient must be coated or formulated in a pharmaceutical composition, digestion of which is prevented. In addition, the pharmaceutical composition may be administered by using any device capable of moving an active material toward a target cell.

A suitable dosage of the pharmaceutical composition may depend on many factors, such as formulation methods, administration methods, ages, body weight, gender, and pathologic conditions of patients, diets, administration time, administration route, excretion speed, and reaction sensitivity. The desirable dose of the pharmaceutical composition may be in the range of about 0.001 to 100 mg/kg for an adult. The term "pharmaceutically effective amount" or "therapeutically effective amount" used herein refers to an amount used in preventing or treating cancer and/or angiogenesis-related diseases.

The pharmaceutical composition may be formulated, with a pharmaceutically acceptable carrier and/or an additive, into a unit or a multiple dosage form by a well-known method in the art. In this regard, the formulation may be a solution in oil or an aqueous medium, a suspension, a syrup, an emulsifying solution, an extract, powder, granules, a tablet, or a capsule, and may further include a dispersing or a stabilizing agent. In addition, the pharmaceutical composition may be administered as an individual drug, or together with other drugs, and may be administered sequentially or simultaneously with pre-existing drugs. The pharmaceutical composition includes the antibody or the antigen-binding fragments thereof, and thus may be formulated as an immunoliposome. The liposome containing the antibody may be prepared using a well-known method in the art. The immunoliposome is a lipid composition including phosphatidylcholine, cholesterol, and polyethyleneglycol-derived phosphatidylethanolamine, and may be prepared by a reverse phase evaporation method. For example, Fab' fragments may be adhered to the liposome through a disulfide exchange reaction. A chemical drug, such as doxorubicin, may also be included in the liposome.

According to an embodiment, the antibody or antibody fragment may act as an antagonist against the c-Met protein.

The term "antagonist" is understood to include all molecules that partially or entirely block, inhibit, and/or neutralize at least one biological activity of their targets (e.g., c-Met). For example, the term "antagonist antibody" refers to an antibody that inhibits or decreases the biological activity of an antigen to which the antibody binds (e.g., c-Met). An antagonist may decrease receptor phosphorylation due to binding receptors to ligands, promote degradation, or may incapacitate or destroy cells that are activated by the ligands. Also, an antagonist may completely block interaction between a receptor and a ligand, or may practically decrease the interaction due to tertiary structure change or down regulation of the receptor.

According to another embodiment, there is provided the anti c-Met antibody or the antigen-binding fragment as defined in the appended claims for use in preventing and/or treating cancer in a subject in need of preventing and/or treating a cancer. The use of the antibody or antibody fragment may comprise administering a pharmaceutically effective amount, and may comprise administering as a pharmaceutical composition formulated with a pharmaceutically acceptable carrier, a diluent, or excipient, as described herein. The use may further include identifying a subject in need of preventing and/or treating a cancer, prior to the administering step.

According to another embodiment, there is provided the anti c-Met antibody or the antigen-binding fragment as defined in the appended claims for use in preventing and/or treating a cancer.

The subject to which anti c-Met antibody or the antigen-binding fragment or the pharmaceutical composition for use in preventing or treating cancer may be administered includes an animal, such as a mammal. For example, the animal may be a human, dog, cat, or mouse.

According to another embodiment of the present invention, there is provided a nucleic acid encoding an antibody or antigen binding fragment thereof as described herein, as well as a nucleic acid encoding any of the foregoing polypeptides or amino acid sequences. The nucleic acid encoding the antibody or antigen binding fragment thereof may be, for example, DNA or RNA and may optionally be incorporated in a vector, such as an expression vector.

According to another embodiment of the present invention, there is provided a cell comprising a nucleic acid encoding an antibody or antigen binding fragment thereof as described herein, as well as a nucleic acid encoding any of the foregoing polypeptides or amino acid sequences.

According to another disclosure, there is disclosed a method of preparing an antibody or antigen binding fragment thereof as described herein, the method comprising expressing a nucleic acid encoding the antibody or antigen binding fragment thereof in a cell.

One or more embodiments of the present invention will now be described in further detail with reference to the following Examples.

### Example 1: Preparation of mouse antibody AbF46 against c-Met

### Immunization of mice

To obtain immunized mice necessary for developing hybridoma cell lines, 100 µg of human c-Met/Fc fusion protein (R&D Systems) and a complete Freund's adjuvant in the same amount were mixed, and the mixture was administered via an intraperitoneal injection to each of five 4 to 6-week-old BALB/c mice (Japan SLC, Inc.). After two weeks, the antigen (half the previously injected amount) was mixed with an incomplete Freund's adjuvant using the same method as described above, and the mixture was administered to each mouse via an intraperitoneal injection. After one week, final boosting was performed, and blood was collected from the tail of each mouse after three days to obtain serum. Then, serum was diluted at 1/1000 with PBS, and an enzyme-linked immunosorbent assay (ELISA) was performed to analyze whether the titer of the antibody recognizing c-Met increased. Afterwards, mice in which a sufficient amount of the antibody was obtained were selected, and a cell fusion process was performed on the selected mice.

### (2) Cell fusion and preparation of hybridoma cells

Three days before a cell fusion experiment, a mixture of 50 µg of PBS and human c-Met/Fc fusion protein was administered via an intraperitoneal injection to each mouse (BALB/c mice; Japan SLC, Inc.). Each immunized mouse was anesthetized, and its spleen located on the left side of the body was then extracted and ground with a mesh to isolate cells, which were mixed with a culture medium (DMEM, GIBCO, Invitrogen) to prepare a spleen cell suspension. The suspension was centrifuged to collect a cell layer. The obtained 1 x 10⁸ spleen cells were mixed with 1 x 10⁸ myeloma cells (Sp2/0), and the mixture was centrifuged to precipitate the cells. The precipitate was slowly dispersed, treated with 1 ml of 45% (w/v) polyethylene glycol (PEG) in DMEM, and maintained at 37°C for one minute before adding 1 ml of DMEM. After introducing additional 10 ml of DMEM for 1 minute, the resultant was maintained in a water bath at 37°C for 5 minutes. The total amount thereof was made to reach 50 ml, and the resultant was centrifuged. The resulting cell precipitate was re-suspended in an isolation medium (HAT medium) at a concentration of 1×10⁵ cells/ml to 2×10⁵ cells/ml. Then, the resultant was distributed to a 96-well plate (0.1 ml per well), which was incubated in a carbon dioxide incubator at 37°C to prepare the hybridoma cells.

### (3) Selection of hybridoma cells that produce monoclonal antibodies against c-Met protein

To select the hybridoma cells that specifically bind to c-Met from the hybridoma cells prepared in operation (2) described above, ELISA was performed to screen for the cells that produced antibodies active against human c-Met/Fc fusion protein and human Fc protein. 50 µl (2 µg/ml) of human c-Met/Fc fusion protein was coated on each well of a microtiter plate, and unreacted antigens were removed by washing. To exclude antibodies binding to Fc, but not to c-Met, the human Fc protein was coated on each well of a different microtiter plate using the same method as above. Then, 50 µl of a hybridoma cell suspension was added to each well of the microtiter plates to react for 1 hour. Then, the microwell plates were washed with a phosphate buffer-tween 20 (TBST) solution to remove unreacted culture medium. Goat anti-mouse IgG-horseradish peroxidase (IgG-HRP) was added thereto, and a reaction was allowed to occur at room temperature for 1 hour, and washing was performed with the TBST solution. Subsequently, a substrate solution (OPD) of peroxidase was added to each well, and the reaction degree was evaluated by measuring the absorption at 450 nm using an ELISA reader. Through this method, hybridoma cell lines that produce antibodies highly specific to the human c-Met protein and not to the human Fc protein were repeatedly selected. A limiting dilution was performed on the obtained hybridoma cell lines to obtain a single clone of hybridoma cell lines producing monoclonal antibodies. The selected hybridoma cell line producing the monoclonal antibody was registered in the Korean Cell Line Bank with accession number KCLRF-BP-00220.

### (4) Production and purification of the monoclonal antibody

The hybridoma cells obtained in operation (3) described above were cultured in a serum free medium to produce monoclonal antibodies and the monoclonal antibodies were purified.

First, the hybridoma cells cultured in 50 ml of culture medium (DMEM) with 10% (w/v) FBS were centrifuged to obtain a cell precipitate, which was washed with 20 ml of PBS more than twice to remove the FBS. Then, 50 ml of DMEM was introduced to resuspend the cell precipitate, and the resultant was incubated in a carbon dioxide incubator at 37°C for 3 days. After centrifugation to remove antibody-producing cells, cell culture including antibodies was isolated and stored at 4°C, or used directly. Antibodies were purified from 50 to 300 ml of the culture using a AKTA purification device (GE Health) equipped with an affinity column (protein G agarose column; Pharmacia, USA), and the purified antibodies were stored by replacing the supernatant with PBS using a filter for protein aggregation (Amicon).

### Example 2: Preparation of chimeric antibody chAbF46 against c-Met

Generally, when a mouse antibody is injected into a human for medical purposes, immunogenicity may often occur. Thus, to reduce the immunogenicity, a chimeric antibody chAbF46, in which the constant region is substituted with the amino acid sequence of a human IgG1 antibody, was prepared from the mouse antibody AbF46 prepared in Example 1.

Genes were synthesized such that nucleic acid sequence corresponding to a heavy chain was EcoRI-signal sequence-VH-Nhel-CH-TGA-Xhol (SEQ ID NO: 38) and nucleic acid sequence corresponding to a light chain was EcoRI-signal sequence-VL- BsiWI-CL-TGA-Xhol (SEQ ID NO: 39). Then, vectors for expression of a chimeric antibody was constructed by cloning a DNA fragment (SEQ ID NO: 38) having the nucleic acid sequence corresponding to the heavy chain in a pOptiVEC™-TOPO TA Cloning Kit included in an OptiCHO™ Antibody Express Kit (Cat No. 12762-019) manufactured by Invitrogen and a DNA fragment (SEQ ID NO: 39) having the nucleic acid sequence corresponding to the light chain in a pcDNA™3.3-TOPO TA Cloning Kit (Cat No. 8300-01) by using restriction enzymes, EcoRI(NEB, R0101S) and Xhol(NEB, R0146S), respectively.

The constructed vectors were amplified using a Qiagen Maxiprep kit (Cat No. 12662), and vectors including the heavy chain and vectors including the light chain were added to 293T cells (2.5 x 10⁷) at a ratio of about 4:1 (about 80 µg:20 µg) with 360 µl of 2 M CaCl₂ and were transfected. Next, the mixture was cultured in a DMEM medium with 10% (w/v) FBS at 37°C in 5% (v/v) CO₂ conditions for 5 hours, and then cultured in a DMEM medium without FBS at 37°C in 5% (v/v) CO₂ conditions for 48 hours.

The cultured cells were centrifuged, and 100 ml of each supernatant was purified using AKTA Prime (GE healthcare). Protein A column (GE healthcare, 17-0405-03) was placed in the AKTA Prime, and the cultured solution was flowed at a flow rate of 5 ml/min and was eluted with IgG elution buffer (Thermo Scientific, 21004). The buffer was replaced with a PBS buffer, and thus a final chimeric antibody (hereinafter, chAbF46) was purified.

### Example 3: Preparation of humanized antibody huAbF46 from chimeric antibody chAbF46

### Heavy chain humanization

For the H1-heavy chain and the H3-heavy chain, the human germline gene most homologous to a VH gene of mouse antibody AbF46 was identified using NCBI Ig Blast. VH3-71 was confirmed to have 83% homology at an amino acid level. CDR-H1, CDR-H2, and CDR-H3 of mouse antibody AbF46 were numbered using Kabat numbering and a CDR portion of mouse antibody AbF46 was introduced in a framework of VH3-71. Amino acids of No. 30 (S→T), No. 48 (V→L), No. 73 (D→N), and No. 78 (T→L) were back-mutated to the amino acid sequence of the original mouse AbF46 antibody, wherein the number of the amino acid is numbered according to Kabat numbering, and thus, the number is common to the VH3-71 and mouse AbF46 antibody. Then, in the H1-heavy chain, the amino acids of No. 83 (R→K) and No. 84 (A→T) were additionally mutated, thereby completing construction of H1-heavy chain (SEQ ID NO: 40) and H3-heavy chain (SEQ ID NO: 41).

For the H4-heavy chain, a framework sequence of a human antibody was obtained, and the VH3 subtype (known to have a sequence similar to the mouse framework sequence of the AbF46 antibody and to be stable) was used to introduce CDR-H1, CDR-H2, and CDR-H3 of mouse antibody AbF46 defined using Kabat numbering. Accordingly, the H4-heavy chain (SEQ ID NO: 42) was constructed.

### (2) Light chain humanization

For the H1-light chain (SEQ ID NO: 43) and the H2-light chain (SEQ ID NO: 44), the human germline gene most homologous to the VL gene of mouse antibody AbF46 was identified using NCBI Ig Blast. VK4-1 was confirmed to have 75% of homology at the amino acid level. CDR-L1, CDR-L2, and CDR-L3 of mouse antibody AbF46 were defined using Kabat numbering and a CDR portion of mouse antibody AbF46 was introduced into a framework of VK4-1. In the H1-light chain, 3 amino acids of No. 36 (Y→H), No. 46 (L→M), and No. 49 (Y→I) were back-mutated. In the H2-light chain, only one amino acid of No. 49 (Y→I) was back-mutated.

For the H3-light chain (SEQ ID NO: 45), the human germline gene most homologous to the VL gene of mouse antibody AbF46 was identified using NCBI Ig Blast. As a result, VK2-40 in addition to VK4-1 (mentioned above) was chosen. Mouse antibodies AbF46 VL and VK2-40 were confirmed to have 61% homology at an amino acid level. CDR-L1, CDR-L2, and CDR-L3 of mouse antibody AbF46 were defined using Kabat numbering and a CDR portion of the mouse antibody AbF46 was introduced into a framework of VK4-1. In the H3-light chain, 3 amino acids of No. 36 (Y→H), No. 46 (L→M), and No. 49 (Y→I) were back-mutated.

For the H4-light chain (SEQ ID NO: 46), a framework sequence of a human antibody was obtained, and the VK1 subtype (conventionally known to be stable) was used to introduce CDR-L1, CDR-L2, and CDR-L3 of mouse antibody AbF46 defined using Kabat numbering. In the H4-light chain, 3 amino acids of No. 36 (Y→H), No. 46 (L→M), and No. 49 (Y→I) were additionally back-mutated.

Then, vectors for expression of the humanized antibody were constructed by cloning DNA fragments (H1-heavy; SEQ ID NO: 47, H3-heavy; SEQ ID NO: 48, and H4-heavy; SEQ ID NO: 49) having the nucleic acid sequence corresponding to the heavy chain in a pOptiVEC™-TOPO TA Cloning Kit included in an OptiCHO™ Antibody Express Kit (Cat No. 12762-019) manufactured by Invitrogen and DNA fragments (H1-light; SEQ ID NO: 50, H2-light; SEQ ID NO: 51, H3-light; SEQ ID NO: 52, and H4-light; SEQ ID NO: 53) having the nucleic acid sequence corresponding to the light chain in a pcDNA™3.3-TOPO TA Cloning Kit (Cat No. 8300-01) by using restriction enzymes, EcoRI(NEB, R0101S) and Xhol(NEB, R0146S), respectively.

The constructed vectors were amplified using a Qiagen Maxiprep kit (Cat No. 12662), and vectors including the heavy chain and vectors including the light chain were added to 293T cells (2.5 x 10⁷) at a ratio of about 4:1 (about 80 µg: 20 µg) with 360 µl of 2 M CaCl₂ and were transfected. Next, the mixture was cultured in a DMEM medium added with 10% (w/v) FBS at 37°C in 5% (v/v) CO₂ conditions for 5 hours, and then cultured in a DMEM medium without FBS at 37°C in 5% (v/v) CO₂ conditions for 48 hours.

The cultured cells were centrifuged, and 100 ml of each supernatant was purified using AKTA Prime (GE healthcare). Protein A column (GE healthcare, 17-0405-03) was placed in the AKTA Prime, and the cultured solution was flowed at a flow rate of 5 ml/min and was eluted with IgG elution buffer (Thermo Scientific, 21004). The buffer was exchanged with a PBS buffer, and thus a final humanized antibody (hereinafter, huAbF46) was purified. The combination of the H4-heavy chain and the H4-light chain of humanized huAbF46 were used hereinafter.

### Example 4: Preparation of scFv library of huAbF46 antibody

Genes for preparing scFv of huAbF46 antibody were designed by using the heavy chain variable region and light chain variable region of huAbF46 antibody. Each of the heavy chain variable region and light chain variable region was designed to have a 'VH-linker-VL' form, in which the linker was designed to have an amino acid sequence of 'GLGGLGGGGSGGGGSGGSSGVGS' (SEQ ID NO: 54). A polynucleotide (SEQ ID NO: 55) encoding scFv of huAbF46 antibody designed as described above was synthesized (Bioneer, Inc.), and a vector for expressing the polynucleotide was represented as SEQ ID NO: 56.

Then, resultants expressed by the vector were analyzed, and c-Met specific binding was identified.

### Example 5: Preparation of library gene for affinity maturation

### Selection of target CDR and preparation of primer

For affinity maturation of huAbF46 antibody, 6 complementarity determining regions (CDRs) were defined by 'Kabat numbering' from the prepared mouse antibody AbF46. CDRs are shown in Table 1.

**Table 1**

| CDR | amino acid sequence |
|---|---|
| CDR-H1 | DYYMS (SEQ ID NO: 1) |
| CDR-H2 | FIRNKANGYTTEYSASVKG (SEQ ID NO: 2) |
| CDR-H3 | DNWFAY (SEQ ID NO: 3) |
| CDR-L1 | KSSQSLLASGNQNNYLA (SEQ ID NO: 10) |
| CDR-L2 | WASTRVS (SEQ ID NO: 11) |
| CDR-L3 | QQSYSAPLT (SEQ ID NO: 12) |

Primers were prepared as follows in order to randomly introduce sequences of CDRs of antibody. According to existing methods of randomly introducing sequences, N codon was used such that bases could be introduced into sites to be mutated at the same rate (25% A, 25% G, 25% C, and 25% T). However, according to the current embodiment, in order to randomly introduce bases into the CDRs of the huAbF46 antibody, 85% of the first and second nucleotides were preserved among three wild-type nucleotides coding amino acids of each CDR, and 5% of each of the other three bases was introduced. In addition, the primer was designed such that the three bases could be introduced into the third nucleotide (33% G, 33% C, and 33% T).

### (2) Preparation of huAbF46 antibody library and identification of binding affinity to c-Met

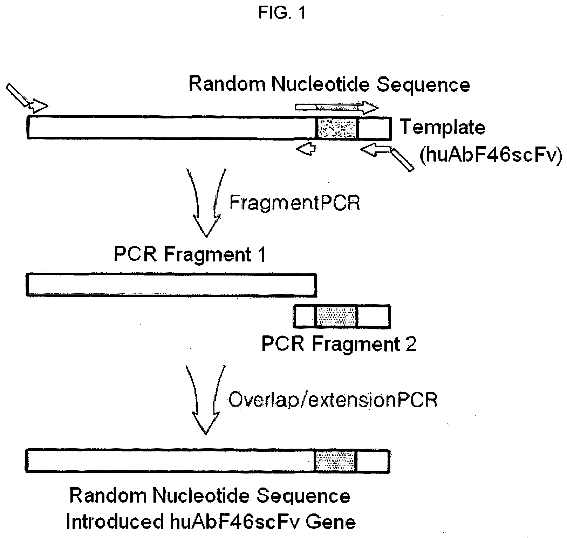

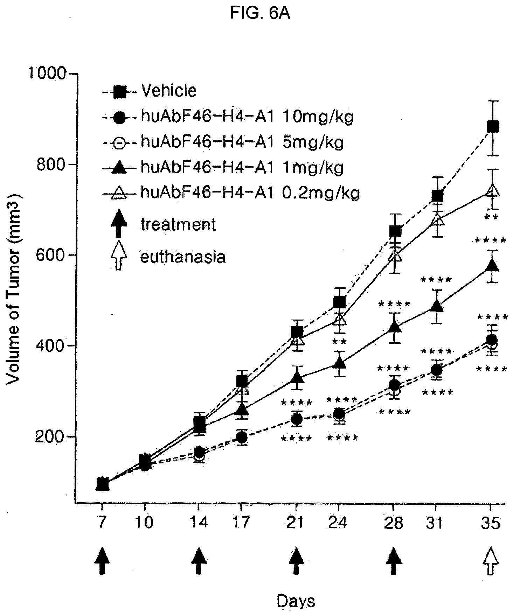

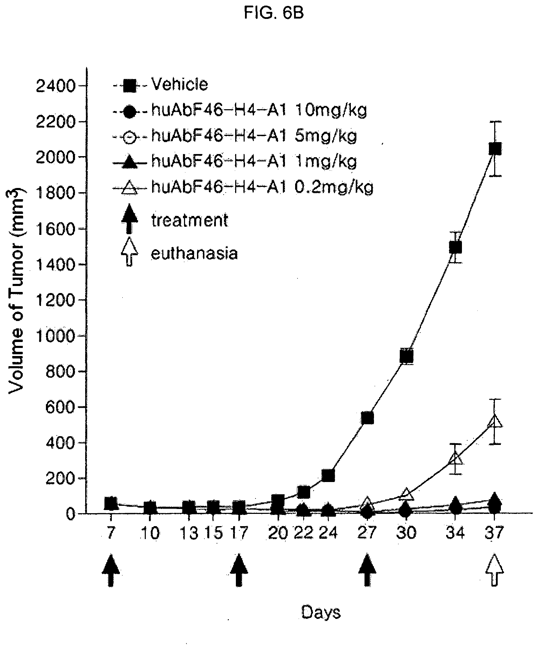

The construction of an antibody gene library by randomly introducing sequences into CDRs was performed using the primer prepared in operation (1) described above. A polynucleotide including nucleic acid sequence encoding scFv of the huAbF46 antibody was used as a template. Two PCR fragments were prepared as shown in FIG. 1 and 6 libraries, respectively targeting the 6 CDRs were constructed by using an overlap extension PCR.

The binding affinities of the wild-type antibody (huAb46) and each antibody derived from the libraries to c-Met were identified. While the binding affinities of most antibodies derived from the libraries to c-Met were lower than that of the wild-type antibody, mutants in which the binding affinity to c-Met was not reduced were identified.

### Example 6: Selection of antibody with improved affinity from the libraries

If the binding affinity of an antibody derived from the libraries to c-Met was improved, the scFv gene sequence from that individual clone was analyzed. The obtained CDR sequences, shown in Table 2 below, were transformed into IgG. Among the clones listed below, 4 types of antibodies produced from L3-1, L3-2, L3-3, and L3-5 were selected and subsequent experiments were performed using these antibodies.

**Table 2**

| Name of clone | Library | CDR sequence |
|---|---|---|
| H11-4 | CDR-H1 | PEYYMS (SEQ ID NO: 22) |
| YC151 | CDR-H1 | PDYYMS (SEQ ID NO: 23) |
| YC193 | CDR-H1 | SDYYMS (SEQ ID NO: 24) |
| YC244 | CDR-H2 | RNNANGNT (SEQ ID NO: 25) |
| YC321 | CDR-H2 | RNKVNGYT (SEQ ID NO: 26) |
| YC354 | CDR-H3 | DNWLSY (SEQ ID NO: 27) |
| YC374 | CDR-H3 | DNWLTY (SEQ ID NO: 28) |
| L1-1 | CDR-L1 | KSSHSLLASGNQNNYLA (SEQ ID NO: 29) |
| L1-3 | CDR-L1 | KSSRSLLSSGNHKNYLA (SEQ ID NO: 30 |
| L1-4 | CDR-L1 | KSSKSLLASGNQNNYLA (SEQ ID NO: 31) |
| L1-12 | CDR-L1 | KSSRSLLASGNQNNYLA (SEQ ID NO: 32 |
| L1-22 | CDR-L1 | KSSHSLLASGNQNNYLA (SEQ ID NO: 33) |
| L2-9 | CDR-L2 | WASKRVS (SEQ ID NO: 34) |
| L2-12 | CDR-L2 | WGSTRVS (SEQ ID NO: 35) |
| L2-16 | CDR-L2 | WGSTRVP (SEQ ID NO: 36) |
| L3-1 | CDR-L3 | QQSYSRPYT (SEQ ID NO: 13) |
| L3-2 | CDR-L3 | GQSYSRPLT (SEQ ID NO: 14) |
| L3-3 | CDR-L3 | AQSYSHPFS (SEQ ID NO: 15) |
| L3-5 | CDR-L3 | QQSYSRPFT (SEQ ID NO: 16) |
| L3-32 | CDR-L3 | QQSYSKPFT (SEQ ID NO: 37) |

### Example 7: Transformation of selected antibodies to IgG

A polynucleotide encoding the heavy chain of the selected 4 types of antibodies consisted of 'EcoRI-signal sequence-VH-Nhel-CH-Xhol' (SEQ ID NO: 38). The amino acids of the heavy chain were not modified after affinity was matured, so the heavy chain of the huAbF46 antibody was used. The hinge region was replaced with a U6-HC7 hinge region (SEQ ID NO: 57), not with the hinge region of human IgG1. A gene of the light chain was designed to have 'EcoRI-signal sequence-VL-BsiWI-CL-XhoI', and polynucleotides (SEQ ID NOS: 58 to 61) encoding light chain variable regions of the selected 4 types of antibodies were synthesized by Bioneer, Inc. Then, vectors for expression of the antibodies were constructed by cloning a DNA fragment (SEQ ID NO: 38) having the nucleic acid sequence corresponding to the heavy chain in a pOptiVEC™-TOPO TA Cloning Kit included in an OptiCHO™ Antibody Express Kit (Cat No. 12762-019) manufactured by Invitrogen and DNA fragments (a DNA fragment including L3-1-derived CDR-L3 (SEQ ID NO: 58), a DNA fragment including L3-2-derived CDR-L3 (SEQ ID NO: 59), a DNA fragment including L3-3-derived CDR-L3 (SEQ ID NO: 60), and a DNA fragment including L3-5-derived CDR-L3 (SEQ ID NO: 61)) corresponding to the light chain in a pcDNA™3.3-TOPO TA Cloning Kit (Cat No. 8300-01) by using a restriction enzyme, EcoRI(NEB, R0101S) and Xhol(NEB, R0146S), respectively.

The constructed vectors were amplified using a Qiagen Maxiprep kit (Cat No. 12662), and vectors including the heavy chain and vectors including the light chain were added to 293T cells (2.5 x 10⁷) at a ratio of about 4:1 (about 80 µg: 20 µg) with 360 µl of 2 M CaCl₂ and were transfected. Next, the mixture was cultured in a DMEM medium with 10% (w/v) FBS at 37°C in 5% (v/v) CO₂ conditions for 5 hours, and then cultured in a DMEM medium without FBS at 37°C in 5% (v/v) CO₂ conditions for 48 hours.

The cultured cells were centrifuged, and 100 ml of each supernatant was purified using AKTA Prime (GE healthcare). Protein A column (GE healthcare, 17-0405-03) was placed in the AKTA Prime, and the cultured solution was flowed at a flow rate of 5 ml/min and was eluted with IgG elution buffer (Thermo Scientific, 21004). The buffer was exchanged with a PBS buffer, and thus 4 types of antibodies having improved affinities (hereinafter, huAbF46-H4-A1, huAbF46-H4-A2, huAbF46-H4-A3, and huAbF46-H4-A5) were purified.

### Example 8: Analysis of binding affinity of selected antibodies

Affinities of the 4 types of antibodies against c-Met antigen prepared in Example 7 were measured by using a Biacore (GE healthcare). About 80 to 110 RU of each antibody was immobilized on a CM5 chip, 9 different concentrations ranging from 0.39 nM to 100 nM of human c-Met protein, as an antigen, were injected at a rate of 30 µl/min to obtain kₒₙ values and k_{off} values as shown in Table 3. Then, K_{D} values were calculated based thereon. A binding affinity of huAbF46 to c-Met antigen was about 2.19 nM, and binding affinities of the four types of antibodies having improved affinities were in a range of 0.06 nM to 0.50 nM (Table 3). This indicates that affinities of the antibodies, which were improved in the form of scFv, were further improved by about 5 times to about 37 times after being transformed to IgG.

**Table 3**

| Antibody | kₒₙ (1/Ms) | k_{off} (1/s) | K_{D} (nM) |
|---|---|---|---|
| huAbF46 | 3.29 x 10⁵ | 7.23 x 10⁻⁴ | 2.19 |
| huAbF46-H4-A1 | 7.39 x 10⁵ | 4.53 x 10⁻⁵ | 0.06 |
| huAbF46-H4-A2 | 5.02 x 10⁵ | 2.53 x 10⁻⁴ | 0.50 |
| huAbF46-H4-A3 | 4.19 x 10⁵ | 1.43 x 10⁻⁴ | 0.34 |
| nuAbF46-H4-A5 | 5.72 x 10⁵ | 2.40 x 10⁻⁴ | 0.42 |

### Example 9: Analysis of in vitro biological activity of selected antibodies having improved affinities

### BrdU assay

A BrdU assay was performed using the antibodies having improved affinities in order to evaluate safety of the antibodies. NCI-H441 (ATCC Cat. # HTB-174), human lung cancer cells, were suspended in a RPMI 1640 medium (Gibco) (2 x 10⁵ cell/ml) to prepare a suspension, and about 100 µl of the suspension was introduced to each well of a 96-well tissue culture plate (Corning, Lowell, MA). The suspension was incubated at 37°C in 5% (v/v) CO₂ conditions for 24 hours, and then the medium was completely removed and replaced with a RPMI 1640 diluted with the antibody. After incubating the suspension at 37°C in 5% (v/v) CO₂ conditions for 21 hours, 5-bromo-2'-deoxyuridine (BrdU) was added and the BrdU assay (Roche, Indianapolis, IN) was performed after a further 3 hours of incubation. After denaturing/fixing cells on the plate, an anti-BrdU antibody was added thereto and a substrate was added after an hour to measure a color reaction using an ELISA spectraMax reader (Molecular Devices, Sunnyvale, CA) at 370 nm. Media was used as a negative control, and an antibody 5D5 (ATCC Cat. # HB11895 separated from hybridoma cells and purified) well known as an agonist was used as a positive control.

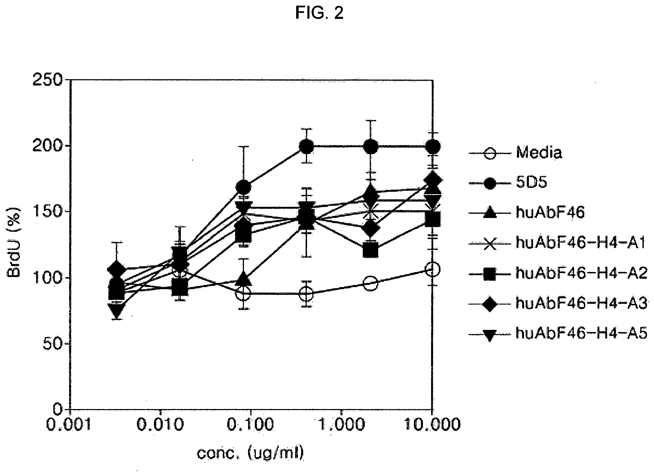

As a result, referring to FIG. 2, among the 4 types of antibodies having improved affinities, agonism side effects of 4 types were reduced. Thus, it was identified that safeties thereof were respectively improved by 25% (huAbF46-H4-A1), 28% (huAbF46-H4-A2), 13% (huAbF46-H4-A3), and 21% (huAbF46-H4-A5) at a concentration of 10 µg/ml.

### (2) In vitro cell proliferation analysis

In order to identify anti-cancer effects of the 4 types of antibodies having improved affinities, as prepared in Example 5, in vitro cell proliferation analysis was performed using MKN45 gastric cancer cells on which c-Met is expressed (Japanese Cancer Research Bank, JCRB, Tokyo, Japan). 1 x 10⁴ MKN45 cells suspended in 50 µl of 5% (w/v) FBS/DMEM culture medium were introduced to each well of a 96-well plate. Then, the cells were either not treated or were treated with 50 µl of the 4 types of antibodies at a concentration of 0.008, 0.04, 0.2, or 1 µg/ml. After incubating for 72 hours, the number of cells was quantified by using a CellTiter-Glo Luminescent Cell Viability Assay Kit (Promega, G7570) with a luminometer (PerkinElmer, 2104 Multilabel reader).

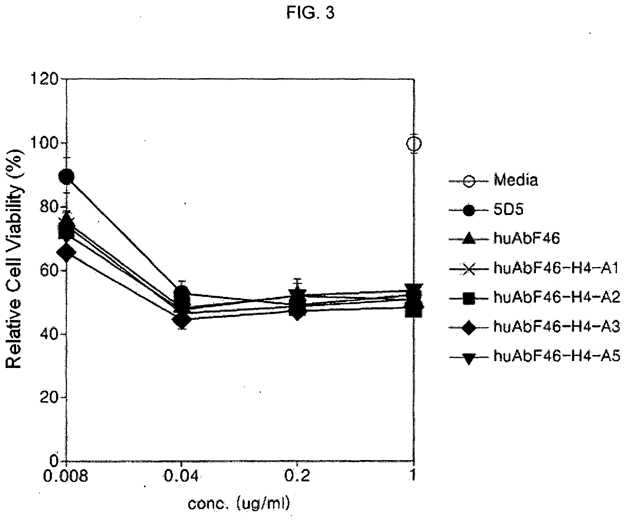

As shown in FIG. 3, relative cell viability of the antibody (huAbF46) in which the affinity was not improved was 77% at the lowest concentration of 0.008 µg/ml, and relative cell viabilities of antibodies having improved affinities, i.e., huAbF46-H4-A1, huAbF46-H4-A2, and huAbF46-H4-A5 were respectively 74, 73, and 72% similar to each other. The relative cell viability of huAbF46-H4-A3, at 66%, was considerably decreased. In addition, at 0.04 µg/ml, where the effect on cell viability is maximized, relative cell viabilities of all of the 4 types of antibodies were equal or less than that of the antibody 5D5 (53%). Accordingly, as a result of improving affinity, efficiency and safety were significantly improved compared to the control.

### (3) Akt phosphorylation

Cellular processes regulated by Akt include cell proliferation, cell survival, cell size control, and response to nutrient availability, intermediary metabolism, angiogenesis, and tissue invasion. All these processes represent characteristics of cancer and many oncoproteins and tumor suppressors intersect in the Akt pathway, finely regulating cellular functions at the interface of signal transduction and classical metabolic regulation. Thus, as the content of phophorylated Akt that is an active form increases, the activity of cancer cells increases. Here, the degree of inhibiting Akt phosphorylation by the 4 types of antibodies having improved affinities was evaluated.

To compare agonism of the 4 types of antibodies having improved affinities, as prepared in Example 5, Caki-1 cells (Korean Cell Line Bank) were used to confirm the degree of Akt phosphorylation. Mouse IgG was used as a negative control, and antibody 5D5 (a known agonist) was used as a positive control.

2 x 10⁵ cells/ml of Caki-1 cells were introduced to a 96-well plate, and after 24 hours, each of 5 µg/ml of the antibodies was treated in serum free medium for 30 minutes. Lysis of the cells treated with the antibodies was performed and the degree of Akt phosphorylation was measured using a PathScan phospho-AKT1 (Ser473) chemiluminescent Sandwich ELISA kit (Cell Signaling, cat. no #7134S).

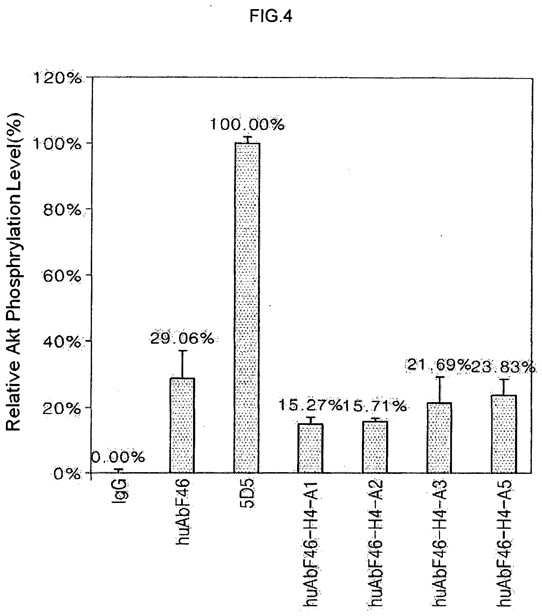

As shown in FIG. 4, it was identified that the degree of inhibiting Akt phosphorylation of all of the 4 types of antibodies was improved. In particular, the degrees of Akt phosphorylation of huAbF46-H4-A1 and huAbF46-H4-A2 were 15.27% and 15.71%, respectively, which were about 49% of that (29.06%) before affinity was improved (huAbF46). Thus, it was identified that safety of the affinity maturated antibodies was considerably improved. In contrast, antibody 5D5 exhibits very high relative Akt phosphorylation level (100%), indicating that antibody 5D5 shows a very high agonism and very low safety.

### (4) Identification of degree of degradation of c-Met

In order to identify anti-cancer effects of the 4 types of antibodies having improved affinities, as prepared in Example 5, the degree of degradation of c-Met bound to the antibodies was evaluated. A relative total amount of c-Met was obtained by measuring the change in the total amount of c-Met after the antibody binds to c-Met to degrade c-Met via internalization, and thus the efficacy of the antibody was evaluated.

MKN45 cells (2 x 10⁵ cells/ml) and each of the antibodies (5 µg/ml) were simultaneously introduced to a 96-well plate and incubated for 24 hours. Then, lysis of the cells treated with antibodies was performed and the change in the total amount of c-Met was measured using a Human total HGF R/c-Met ELISA KIT (R&D systems, DYC358) and analyzed.

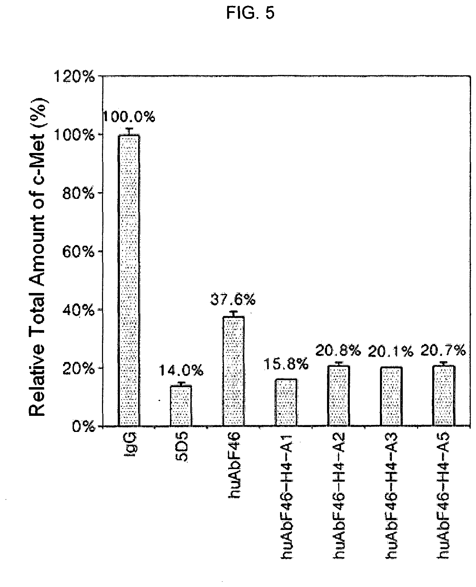

As a result, referring to FIG. 5, it was identified that the degree of degradation of c-Met was improved when cells were treated with the 4 types of antibodies having improved affinities compared to cells treated with the huAbF46 antibody. The degree of degradation of c-Met in cells treated with huAbF46-H4-A1 was increased by about 37% compared to cells treated with huAbF46. The degrees of degradation of c-Met in cells treated with huAbF46-H4-A2, huAbF46-H4-A3, and huAbF46-H4-A5 were increased by about 28% compared to cells treated with huAbF46. As shown in FIGS. 4 and 5, the affinity maturated antibodies show equal or higher degree of degradation of c-Met as well as very higher safety compared to those of antibody 5D5.

### Example 10: Analysis of in vivo biological activity of selected antibodies having improved affinities

In order to identify anti-cancer effects of the 4 types of antibodies having improved affinities, as prepared in Example 5, a decrease in the size of tumor cells in a brain cancer or gastric cancer mouse xenograft model transplanted with U87MG brain cancer cells (Korean Cell Line Bank) or MKN45 gastric cancer cells (Japanese Cancer Research Bank, JCRB, Tokyo, Japan) was observed when the antibodies having improved affinities were administered thereto in vivo.

A 50 µl suspension of 5 x 10⁶ of U87MG brain cancer cells or MKN45 gastric cancer cells was administered via subcutaneous injection to 6 week-old male BALB/C nude mice (Orient Bio Corp.). After 1 week, 12 mice per each group contracted with cancer were randomly selected. A concentration of 10 mg/kg of the 4 types of antibodies having improved affinities was administered via intravenous injection to these mice once a week after tumor cells were formed. Also, as a control, concentrations of 10 mg/kg and 20 mg/kg of mouse AbF46 antibody were administered to the other mice twice a week.

Referring to FIGS. 6A and 6B, in both the U87MG brain cancer and the MKN45 gastric mouse cancer models, tumor growth inhibiting effects were identified.

### Example 11: Preparation of huAbF46-H4-A1 having replaced constant region and/or hinge region

Among the selected 4 types of antibodies, huAbF46-H4-A1 was determined to have the highest binding affinity to c-Met and the lowest degrees of Akt phosphorylation and c-Met differentiation. The hinge region, or the constant region and hinge region, of huAbF46-H4-A1 was replaced.

An antibody including a heavy chain that includes a heavy chain variable region of huAbF46-H4-A1, a U6-HC7 hinge, and a human IgG1 constant region, and a light chain that includes a light chain variable region of huAbF46-H4-A1 and a human kappa constant region was named huAbF46-H4-A1(U6-HC7), an antibody including a heavy chain that includes a heavy chain variable region of huAbF46-H4-A1, a human IgG2 hinge, and a human IgG1 constant region and a light chain that includes a light chain variable region of huAbF46-H4-A1 and a human kappa constant region was named huAbF46-H4-A1 (IgG2 hinge), and an antibody including a heavy chain that includes a heavy chain variable region of huAbF46-H4-A1, a human IgG2 hinge, and a human IgG2 constant region and a light chain that includes a light chain variable region of huAbF46-H4-A1 and a human kappa constant region was named huAbF46-H4-A1 (IgG2 Fc). In addition, in order to increase productivity of the 3 types of antibodies all histidine was replaced with tyrosine at position 36 in the light chain including the human kappa constant region.

In order to prepare the 3 types of antibodies, a polynucleotide (SEQ ID NO: 63) encoding a polypeptide (SEQ ID NO: 62) including the heavy chain variable region of huAbF46-H4-A1, the U6-HC7 hinge region, and the human IgG1 constant region, a polynucleotide (SEQ ID NO: 65) encoding a polypeptide (SEQ ID NO: 64) including the heavy chain variable region of huAbF46-H4-A1, the human IgG2 hinge region, and the human IgG1 constant region, a polynucleotide (SEQ ID NO: 67) encoding a polypeptide (SEQ ID NO: 66) including the heavy chain variable region of huAbF46-H4-A1, the human IgG2 hinge region, and the human IgG2 constant region, and a polynucleotide (SEQ ID NO: 69) encoding a polypeptide (SEQ ID NO: 68) including the light chain variable region of huAbF46-H4-A1 in which histidine is replaced with tyrosine at position 36 and the human kappa constant region were synthesized by Bioneer, Inc. Then, vectors for expression of the antibodies were constructed by cloning a DNA fragment having the nucleic acid sequence corresponding to the heavy chain in a pOptiVEC™-TOPO TA Cloning Kit included in an OptiCHO™ Antibody Express Kit (Cat No. 12762-019) manufactured by Invitrogen, and a DNA fragment having the nucleic acid sequence corresponding to the light chain in a pcDNA™3.3-TOPO TA Cloning Kit (Cat No. 8300-01).

The constructed vectors were amplified using a Qiagen Maxiprep kit (Cat No. 12662), and vectors including the heavy chain and vectors including the light chain were added to 293T cells (2.5 x 10⁷) at a ratio of about 4:1 (about 80 µg:20 µg) with 360 µl of 2 M CaCl₂ and were transfected. Then, the mixture was cultured in a DMEM medium added with 10% (w/v) FBS at 37°C in 5% (v/v) CO₂ conditions for 5 hours, and then cultured in a DMEM medium without FBS at 37°C in 5% (v/v) CO₂ conditions for 48 hours.

The cultured cells were centrifuged, and 100 ml of each supernatant was purified using AKTA Prime (GE healthcare). Protein A column (GE healthcare, 17-0405-03) was placed in the AKTA Prime, and the cultured solution was flowed at a flow rate of 5 ml/min and was eluted with IgG elution buffer (Thermo Scientific, 21004). The buffer was exchanged with a PBS buffer, and 3 types of antibodies (huAbF46-H4-A1(U6-HC7), huAbF46-H4-A1 (IgG2 hinge), and huAbF46-H4-A1(IgG2 Fc)) were purified.

In addition, another light chain (SEQ ID NO: 73) was prepared by replacing the amino acid residue, serine, at the position 27e (according to kabat numbering) of the light chain variable region of huAbF46-H4-A1 with tryptophan. Then, an antibody including the prepared light chain and the heavy chain of huAbF46-H4-A1 (IgG2 Fc) was prepared as described above, and named as L3-11Y. The binding affinity of the L3-11Y antibody was measured according to the method described in Example 8, and the measured binding affinity (K_{D} (nM)) was less than 0.01 (<0.01).

### Example 12: Analysis of in vitro biological activity of huAbF46-H4-A1 having replaced constant region and/or hinge region

### In vitro cell proliferation analysis

In order to identify anti-cancer effects of the three types of antibodies prepared in Example 11, in vitro cell proliferation analysis was performed using MKN45 gastric cancer cells having c-Met on the cell membrane (Japanese Cancer Research Bank, JCRB, Tokyo, Japan). 1 x 10⁴ MKN45 cells suspended in 50 µl of 5% (w/v) FBS/DMEM culture medium were introduced to each well of a 96-well plate. Then, the cells were either not treated or were treated with 50 µl of the 3 types of antibodies at a concentration of 0.008, 0.04, 0.2, or 1 µg/ml. After incubating for 72 hours, the number of cells was quantified by using a CellTiter-Glo Luminescent Cell Viability Assay Kit (Promega, G7570) with a luminometer (PerkinElmer, 2104 Multilabel reader).

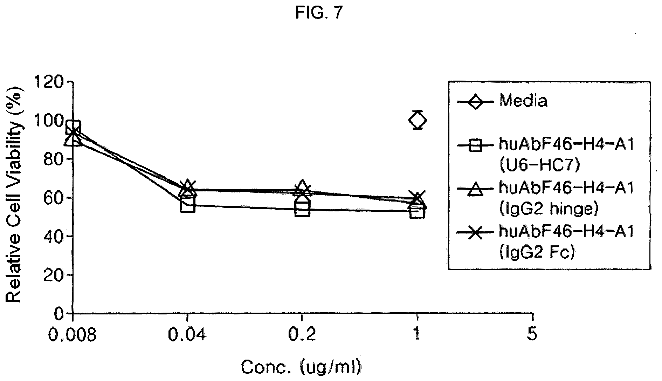

As shown in FIG. 7, when the 3 types of antibodies, huAbF46-H4-A1 (U6-HC7), huAbF46-H4-A1 (IgG2 hinge), and huAbF46-H4-A1 (IgG2 Fc), were treated at a concentration of 0.04 µg/ml or less, the relative cell viability was about 60%.

### (2) Akt phosphorylation

To compare agonism of the 3 types of antibodies having improved affinities, as prepared in Example 11, Caki-1 cells (Korean Cell Line Bank) were used to confirm the degree of Akt phosphorylation. Mouse IgG was used as a negative control, and a 5D5 antibody (a known agonist) was used as a positive control.

2 x 10⁵ cells/ml of Caki-1 cells were introduced to a 96-well plate, and after 24 hours, each of 5 µg/ml of the antibodies was treated in serum free medium for 30 minutes. Lysis of the cells treated with antibodies was performed and a degree of Akt phosphorylation inhibition was measured using a PathScan phospho-AKT1 (Ser473) a chemiluminescent Sandwich ELISA kit (Cell Signaling, cat. no #7134S).

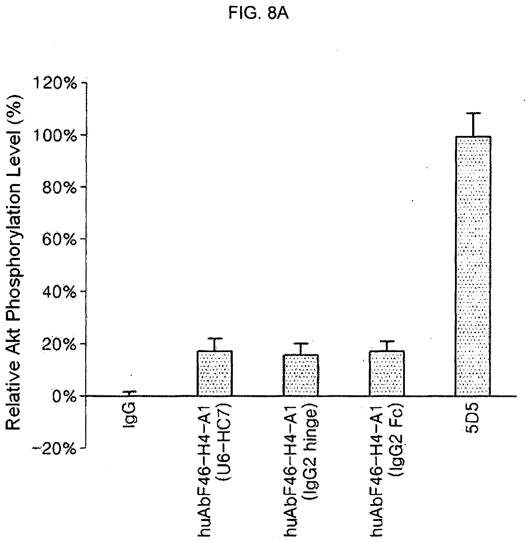

The obtained results are shown in FIG. 8A. As shown in FIG. 8A, the degrees of inhibiting Akt phosphorylation of all of the 3 types of antibodies were 18% or less. Thus, it was identified that safety was considerably improved.

In addition, the degrees of Akt phosphorylation inhibition of huAbF46-H4-A1(IgG2 Fc) and L3-11Y were also measured according the above method. The obtained results are shown in FIG. 8B. As shown in FIG. 8B, L3-11Y exhibits an equal or higher activity of Akt phosphorylation inhibition compared to that of huAbF46-H4-A1(IgG2 Fc).

### (3) Identification of degree of degradation of c-Met

In order to identify anti-cancer effects of the 3 types of antibodies having improved affinities, as prepared in Example 11, the degree of degradation of c-Met bound to the antibody was evaluated. MKN45 cells (2 x 10⁵ cells/ml) and each of the antibodies (5 µg/ml) were simultaneously introduced to a 96-well plate and incubated for 24 hours. Then, lysis of the cells treated with antibodies was performed and a change of the total amount of c-Met was measured using a Human total HGF R/c-Met ELISA KIT (R&D systems, DYC358) and analyzed.

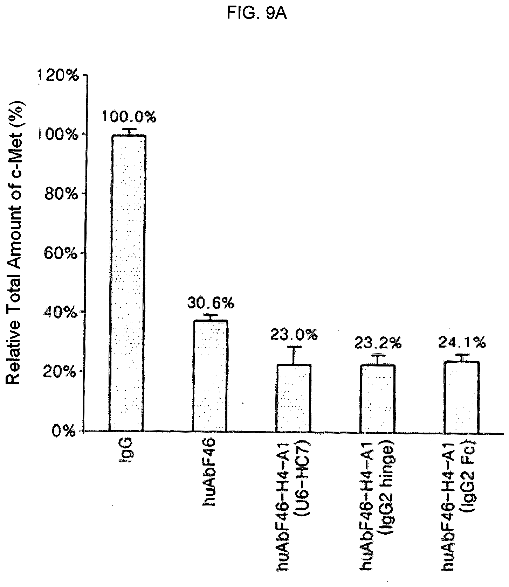

The obtained results are shown in FIG. 9A. As shown in FIG. 9A, it was identified that the degree of degradation of c-Met was improved in cells treated with the 3 types of antibodies having improved affinities compared to cells treated with the huAbF46 antibody.

In addition, the degrees of c-Met degradation of huAbF46-H4-A1 (IgG2 Fc) and L3-11Y were also measured according the above method. The obtained results are shown in FIG. 9B. As shown in FIG. 9B, L3-11Y exhibits an approximately equal activity of c-Met degradation compared to that of huAbF46-H4-A1 (IgG2 Fc).

### Example 13: Analysis of in vivo biological activity of huAbF46-H4-A1 having replaced constant region and/or hinge region

In order to identify anti-cancer effects of the 3 types of antibodies having improved affinities, as prepared in Example 11, a decrease in the size of tumor cells in a brain cancer or gastric cancer mouse xenograft model transplanted with U87MG brain cancer cells (Korean Cell Line Bank) or MKN45 gastric cancer cells (Japanese Cancer Research Bank, JCRB, Tokyo, Japan) was observed when the antibodies having improved affinities were administered in vivo.

A 50 µl suspension of 5 x 10⁶ of U87MG brain cancer cells or MKN45 gastric cancer cells was administered via subcutaneous injection to 6 week-old male BALB/C nude mice (Orient Bio Corp.). After 1 week, 12 mice per each group contracted with cancer were randomly selected. A concentration of 10 mg/kg of the 3 types of antibodies having improved affinities was administered via intravenous injection to these mice once a week after tumor cells were formed. Also, as a control, concentrations of 10 mg/kg and 20 mg/kg of mouse AbF46 antibody were administered to the other mice twice a week.

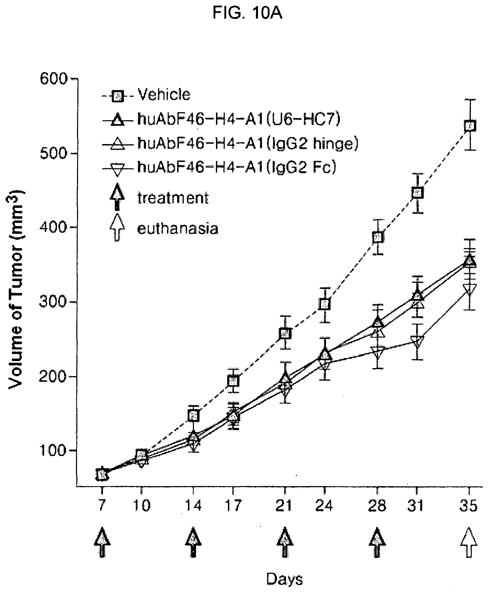

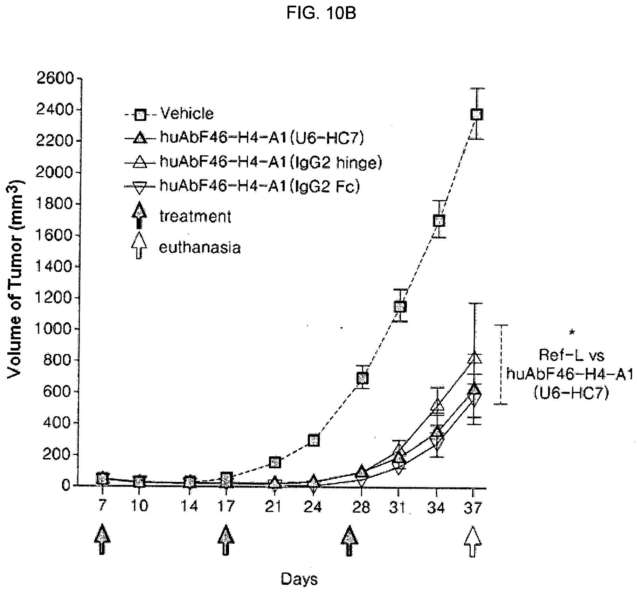

Referring to FIGS. 10A and 10B, in both of the U87MG brain cancer or MKN45 gastric mouse cancer models, tumor growth inhibiting effects were identified.

As described above, according to the anti c-Met antibody and the pharmaceutical composition for preventing or treating cancer including the same according to one or more embodiments of the present invention, cancer may be effectively prevented or treated.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description.

## Claims

1. An anti c-Met antibody or antigen-binding fragment thereof comprising:
a heavy chain variable region comprising the following heavy chain complementarity determining regions (CDRs): a) CDR-H1 having an amino acid sequence of SEQ ID NO: 1, b) CDR-H2 having an amino acid sequence of SEQ ID NO: 2, and c) CDR-H3 having an amino acid sequence of SEQ ID NO: 3; and
a light chain variable region comprising the following light chain CDRs: a) CDR-L1 having an amino acid sequence of SEQ ID NO: 10 or 71, b) CDR-L2 having an amino acid sequence of SEQ ID NO: 11, and c) CDR-L3 having an amino acid sequence of SEQ ID NO: 13, 14, 15, or 16.

2. The anti c-Met antibody or antigen-binding fragment of claim 1, comprising
a heavy chain variable region comprising the following heavy chain complementarity determining regions (CDRs): a) CDR-H1 having an amino acid sequence of SEQ ID NO: 1, b) CDR-H2 having an amino acid sequence of SEQ ID NO: 2, and c) CDR-H3 having an amino acid sequence of SEQ ID NO: 3; and
a light chain variable region comprising the following light chain CDRs:
a) CDR-L1 having an amino acid sequence of SEQ ID NO: 10, b) CDR-L2 having an amino acid sequence of SEQ ID NO: 11, and c) CDR-L3 having an amino acid sequence of SEQ ID NO: 13, 14, 15, or 16.

3. The anti c-Met antibody or antigen-binding fragment of claim 1, comprising:
a heavy chain variable region comprising the following heavy chain complementarity determining regions (CDRs): a) CDR-H1 having an amino acid sequence of SEQ ID NO: 1, b) CDR-H2 having an amino acid sequence of SEQ ID NO: 2, and c) CDR-H3 having an amino acid sequence of SEQ ID NO: 3; and
a light chain variable region comprising the following light chain CDRs:
a) CDR-L1 having an amino acid sequence of SEQ ID NO: 71, b) CDR-L2 having an amino acid sequence of SEQ ID NO: 11, and c) CDR-L3 having an amino acid sequence of SEQ ID NO: 13.

4. The anti c-Met antibody or antigen-binding fragment of any one of claims 1 to 3, wherein the heavy chain variable region has an amino acid sequence of SEQ ID NO: 17, and the light chain variable region has an amino acid sequence of SEQ ID NO: 18, 19, 20, 21, or 72.

5. The anti c-Met antibody or antigen-binding fragment of any one of claims 1 to 3, comprising
(i) a heavy chain comprising an amino acid sequence from 18^{th} to 462^{nd} of SEQ ID NO: 62, an amino acid sequence from 18^{th} to 461^{st} of SEQ ID NO: 64, or amino acid sequence from 18^{th} to 460^{th} of SEQ ID NO: 66, and a light chain comprising an amino acid sequence from 21^{st} to 240^{th} of SEQ ID NO: 68;
(ii) a heavy chain comprising an amino acid sequence from 18^{th} to 462^{nd} of SEQ ID NO: 62, an amino acid sequence from 18^{th} to 461^{st} of SEQ ID NO: 64, or amino acid sequence from 18^{th} to 460^{th} of SEQ ID NO: 66, and a light chain comprising an amino acid sequence from 21^{st} to 240^{th} of SEQ ID NO: 70; or
(iii) a heavy chain comprising an amino acid sequence from 18^{th} to 462^{nd} of SEQ ID NO: 62, an amino acid sequence from 18^{th} to 461^{st} of SEQ ID NO: 64, or amino acid sequence from 18^{th} to 460^{th} of SEQ ID NO: 66, and a light chain comprising an amino acid sequence of SEQ ID NO: 73.

6. The anti c-Met antibody or antigen-binding fragment of any of claims 1-5, wherein the antibody or antigen-binding fragment is a monoclonal antibody, a mouse-derived antibody, a mouse-human chimeric antibody, a humanized antibody.

7. The anti c-Met antibody or antigen-binding fragment of any of claims 1-6, wherein the antigen-binding fragment is scFv, (scFv)₂, Fab, Fab', or F(ab')₂.

8. A pharmaceutical composition comprising the anti c-Met antibody or antigen-binding fragment of any of claims 1-7 and a pharmaceutically acceptable carrier, a diluent, or an excipient.

9. The pharmaceutical composition of claim 8 for use in preventing or treating cancer.

10. The pharmaceutical composition for the use of claim 9, wherein the cancer is squamous cell carcinoma, small-cell lung cancer, non-small-cell lung cancer, adenocarcinoma of the lung, squamous cell carcinoma of the lung, peritoneal carcinoma, skin cancer, skin or intraocular melanoma, colorectal cancer, cancer near the anus, esophagus cancer, small intestinal tumor, endocrine gland cancer, parathyroid cancer, adrenal cancer, soft-tissue sarcoma, urethral cancer, chronic or acute leukemia, lymphocytic lymphoma, hepatoma, gastrointestinal cancer, pancreatic cancer, glioblastoma, cervical cancer, ovarian cancer, liver cancer, bladder cancer, hepatocellular adenoma, breast cancer, colon cancer, large intestine cancer, endometrial carcinoma or uterine carcinoma, salivary gland tumor, kidney cancer, prostate cancer, vulvar cancer, thyroid cancer, or a head or neck cancer.

11. The anti c-Met antibody or antigen-binding fragment of any of claims 1-7 for use in preventing or treating cancer.

12. A polynucleotide encoding the anti c-Met antibody or antibody fragment of any of claims 1-7, optionally in a vector.

13. A cell comprising the polynucleotide of claim 12.

## Patentansprüche

1. Anti-c-Met-Antikörper oder Antigen-bindendes Fragment davon umfassend:
eine variable Region der schweren Kette umfassend die folgenden Regionen (CDRs), die die Komplementarität der schweren Kette bestimmen: a) CDR-H1, die eine Aminosäuresequenz von SEQ ID NR: 1 aufweist, b) CDR-H2, die eine Aminosäuresequenz von SEQ ID NR: 2 aufweist, und c) CDR-H3, die eine Aminosäuresequenz von SEQ ID NR: 3 aufweist; und
eine variable Region der leichten Kette umfassend die folgenden CDRs der leichten Kette:
a) CDR-L1, die eine Aminosäuresequenz von SEQ ID NR: 10 oder 71 aufweist, b) CDR-L2, die eine Aminosäuresequenz von SEQ ID NR: 11 aufweist, und c) CDR-L3, die eine Aminosäuresequenz von SEQ ID NR: 13, 14, 15, oder 16 aufweist.

2. Anti-c-Met-Antikörper oder Antigen-bindendes Fragment nach Anspruch 1, umfassend eine variable Region der schweren Kette umfassend die folgenden Regionen (CDRs), die die Komplementarität der schweren Kette bestimmen: a) CDR-H1, die eine Aminosäuresequenz von SEQ ID NR: 1 aufweist, b) CDR-H2, die eine Aminosäuresequenz von SEQ ID NR: 2 aufweist, und c) CDR-H3, die eine Aminosäuresequenz von SEQ ID NR: 3 aufweist; und
eine variable Region der leichten Kette umfassend die folgenden CDRs der leichten Kette:
a) CDR-L1, die eine Aminosäuresequenz von SEQ ID NR: 10 aufweist, b) CDR-L2, die eine Aminosäuresequenz von SEQ ID NR: 11 aufweist, und c) CDR-L3, die eine Aminosäuresequenz von SEQ ID NR: 13, 14, 15, oder 16 aufweist.

3. Anti-c-Met-Antikörper oder Antigen-bindendes Fragment nach Anspruch 1, umfassend:
eine variable Region der schweren Kette umfassend die folgenden Regionen (CDRs), die die Komplementarität der schweren Kette bestimmen: a) CDR-H1, die eine Aminosäuresequenz von SEQ ID NR: 1 aufweist, b) CDR-H2, die eine Aminosäuresequenz von SEQ ID NR: 2 aufweist, und c) CDR-H3, die eine Aminosäuresequenz von SEQ ID NR: 3 aufweist; und
eine variable Region der leichten Kette umfassend die folgenden CDRs der leichten Kette:
a) CDR-L1, die eine Aminosäuresequenz von SEQ ID NR: 71 aufweist, b) CDR-L2, die eine Aminosäuresequenz von SEQ ID NR: 11 aufweist, und c) CDR-L3, die eine Aminosäuresequenz von SEQ ID NR: 13 aufweist.

4. Anti-c-Met-Antikörper oder Antigen-bindendes Fragment nach einem der Ansprüche 1 bis 3, wobei die variable Region der schweren Kette eine Aminosäuresequenz von SEQ ID NR: 17 aufweist, und die variable Region der leichten Kette eine Aminosäuresequenz von SEQ ID NR: 18, 19, 20, 21, oder 72 aufweist.

5. Anti-c-Met-Antikörper oder Antigen-bindendes Fragment nach einem der Ansprüche 1 bis 3, umfassend
(i) eine schwere Kette umfassend eine Aminosäuresequenz von der 18-ten bis 462-ten von SEQ ID NR: 62, eine Aminosäuresequenz von der 18-ten bis 461-ten von SEQ ID NR: 64, oder Aminosäuresequenz von der 18-ten bis 460-ten von SEQ ID NR: 66, und eine leichte Kette umfassend eine Aminosäuresequenz von der 21-ten bis 240-ten von SEQ ID NR: 68;
(ii) eine schwere Kette umfassend eine Aminosäuresequenz von der 18-ten bis 462-ten von SEQ ID NR: 62, eine Aminosäuresequenz von der 18-ten bis 461-ten von SEQ ID NR: 64, oder Aminosäuresequenz von der 18-ten bis 460-ten von SEQ ID NR: 66, und eine leichte Kette umfassend eine Aminosäuresequenz von der 21-ten bis 240-ten von SEQ ID NR: 70; oder
(iii) eine schwere Kette umfassend eine Aminosäuresequenz von der 18-ten bis 462-ten von SEQ ID NR: 62, eine Aminosäuresequenz von der 18-ten bis 461-ten von SEQ ID NR: 64, oder Aminosäuresequenz von der 18-ten bis 460-ten von SEQ ID NR: 66, und eine leichte Kette umfassend eine Aminosäuresequenz von SEQ ID NR: 73.

6. Anti-c-Met-Antikörper oder Antigen-bindendes Fragment nach einem der Ansprüche 1-5, wobei der Antikörper oder das Antigen-bindende Fragment ein monoklonaler Antikörper, ein von der Maus abgeleiteter Antikörper, ein chimärer Maus-Mensch-Antikörper, ein humanisierter Antikörper ist.

7. Anti-c-Met-Antikörper oder Antigen-bindendes Fragment nach einem der Ansprüche 1-6, wobei das Antigen-bindende Fragment scFv, (scFv)₂, Fab, Fab', oder F(ab')₂ ist.

8. Pharmazeutische Zusammensetzung umfassend den Anti-c-Met-Antikörper oder das Antigen-bindende Fragment nach einem der Ansprüche 1-7 und einen pharmazeutisch akzeptablen Träger, ein Verdünnungsmittel, oder einen Hilfsstoff.

9. Pharmazeutische Zusammensetzung nach Anspruch 8 zur Verwendung in der Prävention oder Behandlung von Krebs.

10. Pharmazeutische Zusammensetzung für die Verwendung nach Anspruch 9, wobei der Krebs Plattenepithelkarzinom, Klein-Zell-Lungenkrebs, Nicht-Klein-Zell-Lungenkrebs, Adenokarzinom der Lunge, Plattenepithelkarzinom der Lunge, peritoneales Karzinom, Hautkrebs, Haut- oder intraokulares Melanom, kolorektaler Krebs, Krebs nahe am Anus, Speiseröhrenkrebs, Dünndarmtumor, endokriner Drüsenkrebs, Nebenschilddrüsenkrebs, Nebennierenkrebs, Weichteilsarkom, Harnröhrenkrebs, chronische oder akute Leukämie, lymphozytisches Lymphom, Hepatom, gastrointestinaler Krebs, Bauchspeicheldrüsenkrebs, Glioblastom, Gebährmutterhalskrebs, Eierstockkrebs, Leberkrebs, Blasenkrebs, hepatozelluläres Adenom, Brustkrebs, Darmkrebs, Dickdarmkrebs, Endometriumkarzinom oder Gebährmutterkrebs, Speicheldrüsentumor, Nierenkrebs, Prostatakrebs, Vulvakrebs, Schilddrüsenkrebs, oder ein Kopf- oder Nackenkrebs ist.

11. Anti-c-Met-Antikörper oder Antigen-bindendes Fragment nach einem der Ansprüche 1-7 zur Verwendung in der Prävention oder Behandlung von Krebs.

12. Polynukleotid, das den Anti-c-Met-Antikörper oder das Antikörperfragment nach einem der Ansprüche 1-7 kodiert, optional in einem Vektor.

13. Zelle umfassend das Polynukleotid nach Anspruch 12.

## Revendications

1. Anticorps anti-cMet ou fragment de celui-ci se liant à un antigène comprenant :
une région variable de chaîne lourde comprenant les régions déterminant la complémentarité de chaîne lourde (CDR) suivantes : a) une CDR-H1 ayant une séquence d'acides aminés de la SEQ ID NO : 1, b) une CDR-H2 ayant une séquence d'acides aminés de la SEQ ID NO : 2 et une CDR-H3 ayant une séquence d'acides aminés de la SEQ ID NO : 3 ; et
une région variable de chaîne légère comprenant les CDR de chaîne légère suivantes : a) une CDR-L1 ayant une séquence d'acides aminés de la SEQ ID NO : 10 ou 71, b) une CDR-L2 ayant une séquence d'acides aminés de la SEQ ID NO : 11 et c) une CDR-L3 ayant une séquence d'acides aminés des SEQ ID NO : 13, 14, 15 ou 16.

2. Anticorps anti-cMet ou fragment se liant à un antigène selon la revendication 1, comprenant
une région variable de chaîne lourde comprenant les régions déterminant la complémentarité de chaîne lourde (CDR) suivantes : a) une CDR-H1 ayant une séquence d'acides aminés de la SEQ ID NO : 1, b) une CDR-H2 ayant une séquence d'acides aminés de la SEQ ID NO : 2 et une CDR-H3 ayant une séquence d'acides aminés de la SEQ ID NO : 3 ; et
une région variable de chaîne légère comprenant les CDR de chaîne légère suivantes : a) une CDR-L1 ayant une séquence d'acides aminés de la SEQ ID NO : 10, b) une CDR-L2 ayant une séquence d'acides aminés de la SEQ ID NO : 11 et c) une CDR-L3 ayant une séquence d'acides aminés des SEQ ID NO : 13, 14, 15 ou 16.

3. Anticorps anti-cMet ou fragment se liant à un antigène selon la revendication 1, comprenant :
une région variable de chaîne lourde comprenant les régions déterminant la complémentarité de chaîne lourde (CDR) suivantes : a) une CDR-H1 ayant une séquence d'acides aminés de la SEQ ID NO : 1, b) une CDR-H2 ayant une séquence d'acides aminés de la SEQ ID NO : 2 et une CDR-H3 ayant une séquence d'acides aminés de la SEQ ID NO : 3 ; et
une région variable de chaîne légère comprenant les CDR de chaîne légère suivantes : a) une CDR-L1 ayant une séquence d'acides aminés de la SEQ ID NO : 71, b) une CDR-L2 ayant une séquence d'acides aminés de la SEQ ID NO : 11 et c) une CDR-L3 ayant une séquence d'acides aminés de la SEQ ID NO : 13.

4. Anticorps anti-cMet ou fragment de celui-ci se liant à un antigène selon l'une quelconque des revendications 1 à 3, dans lesquels la région variable de chaîne lourde a une séquence d'acides aminés de la SEQ ID NO : 17, et la région variable de chaîne légère a une séquence d'acides aminés de la SEQ ID NO : 18, 19, 20, 21 ou 72.

5. Anticorps anti-cMet ou fragment se liant à un antigène selon l'une quelconque des revendications 1 à 3, comprenant
(i) une chaîne lourde comprenant une séquence d'acides aminés allant du 18^{e} au 462^{e} de la SEQ ID NO : 62, une séquence d'acides aminés allant du 18^{e} au 461^{e} de la SEQ ID NO : 64, ou une séquence d'acides aminés allant du 18^{e} au 460^{e} de la SEQ ID NO : 66, et une chaîne légère comprenant une séquence d'acides aminés allant du 21^{e} au 240^{e} de la SEQ ID NO : 68 ;
(ii) une chaîne lourde comprenant une séquence d'acides aminés allant du 18^{e} au 462^{e} de la SEQ ID NO : 62, une séquence d'acides aminés allant du 18^{e} au 461^{e} de la SEQ ID NO : 64, ou une séquence d'acides aminés allant du 18^{e} au 460^{e} de la SEQ ID NO : 66, et une chaîne légère comprenant une séquence d'acides aminés allant du 21^{e} au 240^{e} de la SEQ ID NO : 70 ; ou
(iii) une chaîne lourde comprenant une séquence d'acides aminés allant du 18^{e} au 462^{e} de la SEQ ID NO : 62, une séquence d'acides aminés allant du 18^{e} au 461^{e} de la SEQ ID NO : 64, ou une séquence d'acides aminés allant du 18^{e} au 460^{e} de la SEQ ID NO : 66, et une chaîne légère comprenant une séquence d'acides aminés de la SEQ ID NO : 73.

6. Anticorps anti-cMet ou fragment se liant à un antigène selon l'une quelconque des revendications 1 à 5, l'anticorps ou le fragment de celui-ci se liant à un antigène étant un anticorps monoclonal, un anticorps dérivé de souris, un anticorps chimérique souris-humain, un anticorps humanisé.

7. Anticorps anti-cMet ou fragment se liant à un antigène selon l'une quelconque des revendications 1 à 6, le fragment se liant à un antigène étant scFv, (scFv)₂, Fab, Fab', ou F(ab')₂.

8. Composition pharmaceutique comprenant l'anticorps anti-cMet ou le fragment de celui-ci se liant à un antigène selon l'une quelconque des revendications 1 à 7 et un véhicule, un diluant ou un excipient pharmaceutiquement acceptables.

9. Composition pharmaceutique selon la revendication 8 destinée à être utilisée dans la prévention ou le traitement d'un cancer.

10. Composition pharmaceutique destinée à être utilisée selon la revendication 9, le cancer étant un carcinome des cellules squameuses, un cancer du poumon à petites cellules, un cancer du poumon non à petites cellules, un adénocarcinome du poumon, un carcinome des cellules squameuses du poumon, un carcinome du péritoine, un cancer de la peau, un mélanome de la peau ou intraoculaire, un cancer colorectal, un cancer à proximité de l'anus, un cancer de l'oesophage, une tumeur de l'intestin grêle, un cancer des glandes endocrines, un cancer parathyroïdien, un cancer des glandes surrénales, un sarcome des tissus mous, un cancer de l'urètre, une leucémie chronique ou aigüe, un lymphome lymphocytaire, un hépatome, un cancer gastro-intestinal, un cancer du pancréas, un glioblastome, un cancer du col de l'utérus, un cancer de l'ovaire, un cancer du foie, un cancer de la vessie, un adénome hépatocellulaire, un cancer du sein, un cancer du côlon, un cancer du gros intestin, un carcinome de l'endomètre ou un carcinome de l'utérus, une tumeur des glandes salivaires, un cancer du rein, un cancer de la prostate, un cancer de la vulve, un cancer de la thyroïde ou un cancer de la tête ou du cou.

11. Anticorps anti-cMet ou fragment se liant à un antigène selon l'une quelconque des revendications 1 à 7 destinés à être utilisés dans la prévention ou le traitement d'un cancer.

12. Polynucléotide codant pour l'anticorps anti-cMet ou le fragment d'anticorps selon l'une quelconque des revendications 1 à 7, éventuellement dans un vecteur.

13. Cellule comprenant le polynucléotide selon la revendication 12.


### SEQUENCE LISTING

<110> Samsung Electronics Co. Ltd
<120> Anti c-Met antibody and uses thereof
<130> EP93321FZSEpau
<140> EP12838709.9
   <141> 2012-10-05
<150> PCT/KR2012/008089
   <151> 2012-10-05
<150> KR2012-0110584
   <151> 2012-10-05
<150> KR2012-0074691
   <151> 2012-07-09
<150> KR2011-0101293
   <151> 2011-10-05
<160> 73
<170> BiSSAP 1.3.6
<210> 1
   <211> 5
   <212> PRT
   <213> Artificial Sequence
<220>
   <223> heavy chain CDR1 of AbF46
<400> 1
   Asp Tyr Tyr Met Ser
   1 5
<210> 2
   <211> 19
   <212> PRT
   <213> Artificial Sequence
<220>
   <223> heavy chain CDR2 of AbF46
<400> 2
   Phe Ile Arg Asn Lys Ala Asn Gly Tyr Thr Thr Glu Tyr Ser Ala Ser
   1 5 10 15
   Val Lys Gly
<210> 3
   <211> 6
   <212> PRT
   <213> Artificial Sequence
<220>
   <223> heavy chain CDR3 of AbF46
<400> 3
   Asp Asn Trp Phe Ala Tyr
   1 5
<210> 4
   <211> 6
   <212> PRT
   <213> Artificial Sequence
<220>
   <223> heavy chain CDR1 of c-Met antibody
<220>
   <221> UNSURE
   <222> 1
   <223> Xaa is Pro or Ser
<220>
   <221> UNSURE
   <222> 2
   <223> Xaa is Glu or Asp
<400> 4
   Xaa Xaa Tyr Tyr Met Ser
   1 5
<210> 5
   <211> 8
   <212> PRT
   <213> Artificial Sequence
<220>
   <223> heavy chain CDR2 of c-Met antibody
<220>
   <221> UNSURE
   <222> 3
   <223> Xaa is Asn or Lys
<220>
   <221> UNSURE
   <222> 4
   <223> Xaa is Ala or Val
<220>
   <221> UNSURE
   <222> 7
   <223> Xaa is Asn or Thr
<400> 5
   Arg Asn Xaa Xaa Asn Gly Xaa Thr
   1               5
<210> 6
   <211> 6
   <212> PRT
   <213> Artificial Sequence
<220>
   <223> heavy chain CDR3 of c-Met antibody
<220>
   <221> UNSURE
   <222> 5
   <223> Xaa is Ser or Thr
<400> 6
   Asp Asn Trp Leu Xaa Tyr
   1               5
<210> 7
   <211> 17
   <212> PRT
   <213> Artificial Sequence
<220>
   <223> light chain CDR1 of c-Met antibody
<220>
   <221> UNSURE
   <222> 4
   <223> Xaa is His, Arg, Gln or Lys
<220>
   <221> UNSURE
   <222> 9
   <223> Xaa is Ser or Trp
<220>
   <221> UNSURE
   <222> 12
   <223> Xaa is His or Gln
<220>
   <221> UNSURE
   <222> 13
   <223> Xaa is Lys or Asn
<400> 7
   Lys Ser Ser Xaa Ser Leu Leu Ala Xaa Gly Asn Xaa Xaa Asn Tyr Leu
   1               5                   10                  15
   Ala
<210> 8
   <211> 7
   <212> PRT
   <213> Artificial Sequence
<220>
   <223> light chain CDR2 of c-Met antibody
<220>
   <221> UNSURE
   <222> 2
   <223> Xaa is Ala or Gly
<220>
   <221> UNSURE
   <222> 4
   <223> Xaa is Thr or Lys
<220>
   <221> UNSURE
   <222> 7
   <223> Xaa is Ser or Pro
<400> 8
   Trp Xaa Ser Xaa Arg Val Xaa
   1               5
<210> 9
   <211> 9
   <212> PRT
   <213> Artificial Sequence
<220>
   <223> light chain CDR3 of c-Met antibody
<220>
   <221> UNSURE
   <222> 1
   <223> Xaa is Gly, Ala or Gln
<220>
   <221> UNSURE
   <222> 6
   <223> Xaa is Arg, His, Ser, Ala, Gly or Lys
<220>
   <221> UNSURE
   <222> 8
   <223> Xaa is Leu, Tyr, Phe or Met
<400> 9
   Xaa Gln Ser Tyr Ser Xaa Pro Xaa Thr
   1               5
<210> 10
   <211> 17
   <212> PRT
   <213> Artificial Sequence
<220>
   <223> light chain CDR1 of AbF46
<400> 10
   Lys Ser Ser Gln Ser Leu Leu Ala Ser Gly Asn Gln Asn Asn Tyr Leu
   1               5                   10                  15
   Ala
<210> 11
   <211> 7
   <212> PRT
   <213> Artificial Sequence
<220>
   <223> light chain CDR2 of AbF46
<400> 11
   Trp Ala Ser Thr Arg Val Ser
   1               5
<210> 12
   <211> 9
   <212> PRT
   <213> Artificial Sequence
<220>
   <223> light chain CDR3 of AbF46
<400> 12
   Gln Gln Ser Tyr Ser Ala Pro Leu Thr
   1               5
<210> 13
   <211> 9
   <212> PRT
   <213> Artificial Sequence
<220>
   <223> CDR-L3 derived from L3-1 clone
<400> 13
   Gln Gln Ser Tyr Ser Arg Pro Tyr Thr
   1 5
<210> 14
   <211> 9
   <212> PRT
   <213> Artificial Sequence
<220>
   <223> CDR-L3 derived from L3-2 clone
<400> 14
   Gly Gln Ser Tyr Ser Arg Pro Leu Thr
   1 5
<210> 15
   <211> 9
   <212> PRT
   <213> Artificial Sequence
<220>
   <223> CDR-L3 derived from L3-3 clone
<400> 15
   Ala Gln Ser Tyr Ser His Pro Phe Ser
   1 5
<210> 16
   <211> 9
   <212> PRT
   <213> Artificial Sequence
<220>
   <223> CDR-L3 derived from L3-5 clone
<400> 16
   Gln Gln Ser Tyr Ser Arg Pro Phe Thr
   1 5
<210> 17
   <211> 117
   <212> PRT
   <213> Artificial Sequence
<220>
   <223> heavy chain variable region of anti c-Met humanized antibody(huAbF46-H4)
<400> 17
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Thr Asp Tyr
               20                  25                  30
   Tyr Met Ser Trp Val Arg Gln Ala Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser
           115
<210> 18
   <211> 114
   <212> PRT
   <213> Artificial Sequence
<220>
   <223> light chain variable region of anti c-Met humanized antibody(huAbF46-H4)
<400> 18
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
   65                  70                  75                  80
   Ile Ser Ser Leu Gln Pro Glu Asp Phe Ala Thr Tyr Tyr Cys Gln Gln
                   85                  90                  95
   Ser Tyr Ser Arg Pro Tyr Thr Phe Gly Gln Gly Thr Lys Val Glu Ile
               100                 105                 110
   Lys Arg
<210> 19
   <211> 114
   <212> PRT
   <213> Artificial Sequence
<220>
   <223> light chain variable region of anti c-Met humanized antibody(huAbF46-H4)
<400> 19
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
   65                  70                  75                  80
   Ile Ser Ser Leu Gln Pro Glu Asp Phe Ala Thr Tyr Tyr Cys Gly Gln
                   85                  90                  95
   Ser Tyr Ser Arg Pro Leu Thr Phe Gly Gln Gly Thr Lys Val Glu Ile
               100                 105                 110
   Lys Arg
<210> 20
   <211> 114
   <212> PRT
   <213> Artificial Sequence
<220>
   <223> light chain variable region of anti c-Met humanized antibody(huAbF46-H4)
<400> 20
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
   65                  70                  75                  80
   Ile Ser Ser Leu Gln Pro Glu Asp Phe Ala Thr Tyr Tyr Cys Ala Gln
                   85                  90                  95
   Ser Tyr Ser His Pro Phe Ser Phe Gly Gln Gly Thr Lys Val Glu Ile
               100                 105                 110
   Lys Arg
<210> 21
   <211> 114
   <212> PRT
   <213> Artificial Sequence
<220>
   <223> light chain variable region of anti c-Met humanized antibody(huAbF46-H4)
<400> 21
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
   65                  70                  75                  80
   Ile Ser Ser Leu Gln Pro Glu Asp Phe Ala Thr Tyr Tyr Cys Gln Gln
                   85                  90                  95
   Ser Tyr Ser Arg Pro Phe Thr Phe Gly Gln Gly Thr Lys Val Glu Ile
               100                 105                 110
   Lys Arg
<210> 22
   <211> 6
   <212> PRT
   <213> Artificial Sequence
<220>
   <223> CDR-H1 derived from H11-4 clone
<400> 22
   Pro Glu Tyr Tyr Met Ser
   1               5
<210> 23
   <211> 6
   <212> PRT
   <213> Artificial Sequence
<220>
   <223> CDR-H1 derived from YC151 clone
<400> 23
   Pro Asp Tyr Tyr Met Ser
   1               5
<210> 24
   <211> 6
   <212> PRT
   <213> Artificial Sequence
<220>
   <223> CDR-H1 derived from YC193 clone
<400> 24
   Ser Asp Tyr Tyr Met Ser
   1               5
<210> 25
   <211> 8
   <212> PRT
   <213> Artificial Sequence
<220>
   <223> CDR-H2 derived from YC244 clone
<400> 25
   Arg Asn Asn Ala Asn Gly Asn Thr
   1               5
<210> 26
   <211> 8
   <212> PRT
   <213> Artificial Sequence
<220>
   <223> CDR-H2 derived from YC321 clone
<400> 26
   Arg Asn Lys Val Asn Gly Tyr Thr
   1               5
<210> 27
   <211> 6
   <212> PRT
   <213> Artificial Sequence
<220>
   <223> CDR-H3 derived from YC354 clone
<400> 27
   Asp Asn Trp Leu Ser Tyr
   1               5
<210> 28
   <211> 6
   <212> PRT
   <213> Artificial Sequence
<220>
   <223> CDR-H3 derived from YC374 clone
<400> 28
   Asp Asn Trp Leu Thr Tyr
   1               5
<210> 29
   <211> 17
   <212> PRT
   <213> Artificial Sequence
<220>
   <223> CDR-L1 derived from L1-1 clone
<400> 29
   Lys Ser Ser His Ser Leu Leu Ala Ser Gly Asn Gln Asn Asn Tyr Leu
   1               5                   10                  15
   Ala
<210> 30
   <211> 17
   <212> PRT
   <213> Artificial Sequence
<220>
   <223> CDR-L1 derived from L1-3 clone
<400> 30
   Lys Ser Ser Arg Ser Leu Leu Ser Ser Gly Asn His Lys Asn Tyr Leu
   1               5                   10                  15
   Ala
<210> 31
   <211> 17
   <212> PRT
   <213> Artificial Sequence
<220>
   <223> CDR-L1 derived from L1-4 clone
<400> 31
   Lys Ser Ser Lys Ser Leu Leu Ala Ser Gly Asn Gln Asn Asn Tyr Leu
   1               5                   10                  15
   Ala
<210> 32
   <211> 17
   <212> PRT
   <213> Artificial Sequence
<220>
   <223> CDR-L1 derived from L1-12 clone
<400> 32
   Lys Ser Ser Arg Ser Leu Leu Ala Ser Gly Asn Gln Asn Asn Tyr Leu
   1               5                   10                  15
   Ala
<210> 33
   <211> 17
   <212> PRT
   <213> Artificial Sequence
<220>
   <223> CDR-L1 derived from L1-22 clone
<400> 33
   Lys Ser Ser His Ser Leu Leu Ala Ser Gly Asn Gln Asn Asn Tyr Leu
   1 5 10 15
   Ala
<210> 34
   <211> 7
   <212> PRT
   <213> Artificial Sequence
<220>
   <223> CDR-L2 derived from L2-9 clone
<400> 34
   Trp Ala Ser Lys Arg Val Ser
   1 5
<210> 35
   <211> 7
   <212> PRT
   <213> Artificial Sequence
<220>
   <223> CDR-L2 derived from L2-12 clone
<400> 35
   Trp Gly Ser Thr Arg Val Ser
   1 5
<210> 36
   <211> 7
   <212> PRT
   <213> Artificial Sequence
<220>
   <223> CDR-L2 derived from L2-16 clone
<400> 36
   Trp Gly Ser Thr Arg Val Pro
   1 5
<210> 37
   <211> 9
   <212> PRT
   <213> Artificial Sequence
<220>
   <223> CDR-L3 derived from L3-32 clone
<400> 37
   Gln Gln Ser Tyr Ser Lys Pro Phe Thr
   1 5
<210> 38
   <211> 1416
   <212> DNA
   <213> Artificial Sequence
<220>
   <223> nucleotide sequence of heavy chain of chAbF46
<220>
   <221> misc_feature
   <222> 1..6
   <223> /note="EcoRI restriction site"
<220>
   <221> misc_feature
   <222> 7..66
   <223> /note="signal sequence"
<220>
   <221> misc_feature
   <222> 67..417
   <223> /note="VH - heavy chain variable region"
<220>
   <221> misc_feature
   <222> 418..423
   <223> /note="NdeI restriction site"
<220>
   <221> misc_feature
   <222> 418..1407
   <223> /note="CH - heavy chain constant region"
<220>
   <221> misc_feature
   <222> 1408..1410
   <223> /note="TGA - stop sodon"
<220>
   <221> misc_feature
   <222> 1411..1416
   <223> /note="XhoI restriction site"
<400> 38
   gaattcgccg ccaccatgga atggagctgg gtttttctcg taacactttt aaatggtatc 60
   cagtgtgagg tgaagctggt ggagtctgga ggaggcttgg tacagcctgg gggttctctg 120
   agactctcct gtgcaacttc tgggttcacc ttcactgatt actacatgag ctgggtccgc 180
   cagcctccag gaaaggcact tgagtggttg ggttttatta gaaacaaagc taatggttac 240
   acaacagagt acagtgcatc tgtgaagggt cggttcacca tctccagaga taattcccaa 300
   agcatcctct atcttcaaat ggacaccctg agagctgagg acagtgccac ttattactgt 360
   gcaagagata actggtttgc ttactggggc caagggactc tggtcactgt ctctgcagct 420
   agcaccaagg gcccatcggt cttccccctg gcaccctcct ccaagagcac ctctgggggc 480
   acagcggccc tgggctgcct ggtcaaggac tacttccccg aaccggtgac ggtgtcgtgg 540
   aactcaggcg ccctgaccag cggcgtgcac accttcccgg ctgtcctaca gtcctcagga 600
   ctctactccc tcagcagcgt ggtgaccgtg ccctccagca gcttgggcac ccagacctac 660
   atctgcaacg tgaatcacaa gcccagcaac accaaggtgg acaagaaagt tgagcccaaa 720
   tcttgtgaca aaactcacac atgcccaccg tgcccagcac ctgaactcct ggggggaccg 780
   tcagtcttcc tcttcccccc aaaacccaag gacaccctca tgatctcccg gacccctgag 840
   gtcacatgcg tggtggtgga cgtgagccac gaagaccctg aggtcaagtt caactggtac 900
   gtggacggcg tggaggtgca taatgccaag acaaagccgc gggaggagca gtacaacagc 960
   acgtaccgtg tggtcagcgt cctcaccgtc ctgcaccagg actggctgaa tggcaaggag 1020
   tacaagtgca aggtctccaa caaagccctc ccagccccca tcgagaaaac catctccaaa 1080
   gccaaagggc agccccgaga accacaggtg tacaccctgc ccccatcccg ggaggagatg 1140
   accaagaacc aggtcagcct gacctgcctg gtcaaaggct tctatcccag cgacatcgcc 1200
   gtggagtggg agagcaatgg gcagccggag aacaactaca agaccacgcc tcccgtgctg 1260
   gactccgacg gctccttctt cctctacagc aagctcaccg tggacaagag caggtggcag 1320
   caggggaacg tcttctcatg ctccgtgatg catgaggctc tgcacaacca ctacacgcag 1380
   aagagcctct ccctgtctcc gggtaaatga ctcgag 1416
<210> 39
   <211> 759
   <212> DNA
   <213> Artificial Sequence
<220>
   <223> nucleotide sequence of light chain of chAbF46
<220>
   <221> misc_difference
   <222> 1..6
   <223> /note="EcoRI restriction site"
<220>
   <221> misc_difference
   <222> 7..90
   <223> /note="signal sequence"
<220>
   <221> misc_difference
   <222> 91..432
   <223> /note="VL - light chain variable region"
<220>
   <221> misc_difference
   <222> 430..435
   <223> /note="BsiWI restriction site"
<220>
   <221> misc_difference
   <222> 433..750
   <223> /note="CL - light chain constant region"
<220>
   <221> misc_difference
   <222> 751..753
   <223> /note="stop codon"
<220>
   <221> misc_difference
   <222> 754..759
   <223> /note="XhoI restriction site"
<400> 39
   gaattcacta gtgattaatt cgccgccacc atggattcac aggcccaggt cctcatgttg      60
   ctgctgctat cggtatctgg tacctgtgga gacattttga tgacccagtc tccatcctcc     120
   ctgactgtgt cagcaggaga gaaggtcact atgagctgca agtccagtca gagtctttta     180
   gctagtggca accaaaataa ctacttggcc tggcaccagc agaaaccagg acgatctcct     240
   aaaatgctga taatttgggc atccactagg gtatctggag tccctgatcg cttcataggc     300
   agtggatctg ggacggattt cactctgacc atcaacagtg tgcaggctga agatctggct     360
   gtttattact gtcagcagtc ctacagcgct ccgctcacgt tcggtgctgg gaccaagctg     420
   gagctgaaac gtacggtggc tgcaccatct gtcttcatct tcccgccatc tgatgagcag     480
   ttgaaatctg gaactgcctc tgttgtgtgc ctgctgaata acttctatcc cagagaggcc     540
   aaagtacagt ggaaggtgga taacgccctc caatcgggta actcccagga gagtgtcaca     600
   gagcaggaca gcaaggacag cacctacagc ctcagcagca ccctgacgct gagcaaagca     660
   gactacgaga aacacaaagt ctacgcctgc gaagtcaccc atcagggcct gagctcgccc     720
   gtcacaaaga gcttcaacag gggagagtgt tgactcgag                           759
<210> 40
   <211> 447
   <212> PRT
   <213> Artificial Sequence
<220>
   <223> amino acid sequence of H1-heavy
<400> 40
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Thr Asp Tyr
               20                  25                  30
   Tyr Met Ser Trp Val Arg Gln Ala Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Asn Ser
   65                  70                  75                  80
   Leu Tyr Leu Gln Met Asn Ser Leu Lys Thr Glu Asp Thr Ala Val Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 41
   <211> 447
   <212> PRT
   <213> Artificial Sequence
<220>
   <223> amino acid sequence of H3-heavy
<400> 41
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Thr Asp Tyr
               20                  25                  30
   Tyr Met Ser Trp Val Arg Gln Ala Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Asn Ser
   65                  70                  75                  80
   Leu Tyr Leu Gln Met Asn Ser Leu Arg Ala Glu Asp Thr Ala Val Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
           370                     375                     380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 42
   <211> 447
   <212> PRT
   <213> Artificial Sequence
<220>
   <223> amino acid sequence of H4-heavy
<400> 42
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Thr Asp Tyr
               20                  25                  30
   Tyr Met Ser Trp Val Arg Gln Ala Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 43
   <211> 220
   <212> PRT
   <213> Artificial Sequence
<220>
   <223> amino acid sequence of H1-light
<400> 43
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Leu Leu Ala Ser
               20                  25                  30
   Gly Asn Gln Asn Asn Tyr Leu Ala Trp His Gln Gln Lys Pro Gly Gln
           35                  40                  45
   Pro Pro Lys Met Leu Ile Ile Trp Ala Ser Thr Arg Val Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Ser Tyr Ser Ala Pro Leu Thr Phe Gly Gly Gly Thr Lys Val Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 44
   <211> 220
   <212> PRT
   <213> Artificial Sequence
<220>
   <223> amino acid sequence of H2-light
<400> 44
   Asp Ile Val Met Thr Gln Thr Pro Leu Ser Leu Pro Val Thr Pro Gly
   1               5                   10                  15
   Glu Pro Ala Ser Ile Ser Cys Lys Ser Ser Gln Ser Leu Leu Ala Ser
               20                  25                  30
   Gly Asn Gln Asn Asn Tyr Leu Ala Trp His Leu Gln Lys Pro Gly Gln
           35                  40                  45
   Ser Pro Gln Met Leu Ile Ile Trp Ala Ser Thr Arg Val Ser Gly Val
       50                  55                  60
   Pro Asp Arg Phe Ser Gly Ser Gly Ser Gly Thr Asp Phe Thr Leu Lys
   65                  70                  75                  80
   Ile Ser Arg Val Glu Ala Glu Asp Val Gly Val Tyr Tyr Cys Gln Gln
                   85                  90                  95
   Ser Tyr Ser Ala Pro Leu Thr Phe Gly Gln Gly Thr Lys Leu Glu Leu
   100 105 110
   Lys Arg Thr Val Ala Ala Pro Ser Val Phe Ile Phe Pro Pro Ser Asp
   115 120 125
   Glu Gln Leu Lys Ser Gly Thr Ala Ser Val Val Cys Leu Leu Asn Asn
   130 135 140
   Phe Tyr Pro Arg Glu Ala Lys Val Gln Trp Lys Val Asp Asn Ala Leu
   145 150 155 160
   Gln Ser Gly Asn Ser Gln Glu Ser Val Thr Glu Gln Asp Ser Lys Asp
   165 170 175
   Ser Thr Tyr Ser Leu Ser Ser Thr Leu Thr Leu Ser Lys Ala Asp Tyr
   180 185 190
   Glu Lys His Lys Val Tyr Ala Cys Glu Val Thr His Gln Gly Leu Ser
   195 200 205
   Ser Pro Val Thr Lys Ser Phe Asn Arg Gly Glu Cys
   210 215 220
<210> 45
   <211> 220
   <212> PRT
   <213> Artificial Sequence
<220>
   <223> amino acid sequence of H3-light
<400> 45
   Asp Ile Val Met Thr Gln Ser Pro Asp Ser Leu Ala Val Ser Leu Gly
   1 5 10 15
   Glu Arg Ala Thr Ile Asn Cys Lys Ser Ser Gln Ser Leu Leu Ala Ser
   20 25 30
   Gly Asn Gln Asn Asn Tyr Leu Ala Trp Tyr Gln Gln Lys Pro Gly Gln
   35 40 45
   Pro Pro Lys Leu Leu Ile Ile Trp Ala Ser Thr Arg Val Ser Gly Val
   50 55 60
   Pro Asp Arg Phe Ser Gly Ser Gly Ser Gly Thr Asp Phe Thr Leu Thr
   65 70 75 80
   Ile Ser Ser Leu Gln Ala Glu Asp Val Ala Val Tyr Tyr Cys Gln Gln
   85 90 95
   Ser Tyr Ser Ala Pro Leu Thr Phe Gly Gly Gly Thr Lys Val Glu Ile
   100 105 110
   Lys Arg Thr Val Ala Ala Pro Ser Val Phe Ile Phe Pro Pro Ser Asp
   115 120 125
   Glu Gln Leu Lys Ser Gly Thr Ala Ser Val Val Cys Leu Leu Asn Asn
   130 135 140
   Phe Tyr Pro Arg Glu Ala Lys Val Gln Trp Lys Val Asp Asn Ala Leu
   145 150 155 160
   Gln Ser Gly Asn Ser Gln Glu Ser Val Thr Glu Gln Asp Ser Lys Asp
   165 170 175
   Ser Thr Tyr Ser Leu Ser Ser Thr Leu Thr Leu Ser Lys Ala Asp Tyr
   180 185 190
   Glu Lys His Lys Val Tyr Ala Cys Glu Val Thr His Gln Gly Leu Ser
   195 200 205
   Ser Pro Val Thr Lys Ser Phe Asn Arg Gly Glu Cys
   210 215 220
<210> 46
   <211> 219
   <212> PRT
   <213> Artificial Sequence
<220>
   <223> amino acid sequence of H4-light
<400> 46
   Asp Ile Gln Met Thr Gln Ser Pro Ser Ser Leu Ser Ala Ser Val Gly
   1 5 10 15
   Asp Arg Val Thr Ile Thr Cys Lys Ser Ser Gln Ser Leu Leu Ala Ser
   20 25 30
   Gly Asn Gln Asn Asn Tyr Leu Ala Trp His Gln Gln Lys Pro Gly Lys
   35 40 45
   Ala Pro Lys Met Leu Ile Ile Trp Ala Ser Thr Arg Val Ser Gly Val
   50 55 60
   Pro Ser Arg Phe Ser Gly Ser Gly Ser Gly Thr Asp Phe Thr Leu Thr
   65 70 75 80
   Ile Ser Ser Leu Gln Pro Glu Asp Phe Ala Thr Tyr Tyr Cys Gln Gln
   85 90 95
   Ser Tyr Ser Ala Pro Leu Thr Phe Gly Gln Gly Thr Lys Val Glu Ile
   100 105 110
   Lys Arg Thr Val Ala Ala Pro Ser Val Phe Ile Phe Pro Pro Ser Asp
   115 120 125
   Glu Gln Leu Lys Ser Gly Thr Ala Ser Val Val Cys Leu Leu Asn Asn
   130 135 140
   Phe Tyr Pro Arg Glu Ala Lys Val Gln Trp Lys Val Asp Asn Ala Leu
   145 150 155 160
   Gln Ser Gly Asn Ser Gln Glu Ser Val Thr Glu Gln Asp Ser Lys Asp
   165 170 175
   Ser Thr Tyr Ser Leu Ser Ser Thr Leu Thr Leu Ser Lys Ala Asp Tyr
   180 185 190
   Glu Lys His Lys Val Tyr Ala Cys Glu Val Thr His Gln Gly Leu Ser
   195 200 205
   Ser Pro Val Thr Lys Ser Phe Asn Arg Gly Glu
   210 215
<210> 47
   <211> 1350
   <212> DNA
   <213> Artificial Sequence
<220>
   <223> nucleotide sequence of H1-heavy
<400> 47
   gaggtgcagc tggtggagtc tgggggaggc ttggtccagc ctggagggtc cctgagactc 60
   tcctgtgcag cctctggatt caccttcact gactactaca tgagctgggt ccgccaggct 120
   ccagggaagg ggctggagtg gttgggcttt attagaaaca aagctaacgg ttacaccaca 180
   gaatacagtg cgtctgtgaa aggcagattc accatctcaa gagataattc aaagaactca 240
   ctgtatctgc aaatgaacag cctgaaaacc gaggacacgg ccgtgtatta ctgtgctaga 300
   gataactggt ttgcttactg gggtcaagga accctggtca ccgtctcctc ggctagcacc 360
   aagggcccat cggtcttccc cctggcaccc tcctccaaga gcacctctgg gggcacagcg 420
   gccctgggct gcctggtcaa ggactacttc cccgaaccgg tgacggtgtc gtggaactca 480
   ggcgccctga ccagcggcgt gcacaccttc ccggctgtcc tacagtcctc aggactctac 540
   tccctcagca gcgtggtgac cgtgccctcc agcagcttgg gcacccagac ctacatctgc 600
   aacgtgaatc acaagcccag caacaccaag gtggacaaga aagttgagcc caaatcttgt 660
   gacaaaactc acacatgccc accgtgccca gcacctgaac tcctgggggg accgtcagtc 720
   ttcctcttcc ccccaaaacc caaggacacc ctcatgatct cccggacccc tgaggtcaca 780
   tgcgtggtgg tggacgtgag ccacgaagac cctgaggtca agttcaactg gtacgtggac 840
   ggcgtggagg tgcataatgc caagacaaag ccgcgggagg agcagtacaa cagcacgtac 900
   cgtgtggtca gcgtcctcac cgtcctgcac caggactggc tgaatggcaa ggagtacaag 960
   tgcaaggtct ccaacaaagc cctcccagcc cccatcgaga aaaccatctc caaagccaaa 1020
   gggcagcccc gagaaccaca ggtgtacacc ctgcccccat cccgggagga gatgaccaag 1080
   aaccaggtca gcctgacctg cctggtcaaa ggcttctatc ccagcgacat cgccgtggag 1140
   tgggagagca atgggcagcc ggagaacaac tacaagacca cgcctcccgt gctggactcc 1200
   gacggctcct tcttcctcta cagcaagctc accgtggaca agagcaggtg gcagcagggg 1260
   aacgtcttct catgctccgt gatgcatgag gctctgcaca accactacac gcagaagagc 1320
   ctctccctgt ctccgggtaa atgactcgag 1350
<210> 48
   <211> 1350
   <212> DNA
   <213> Artificial Sequence
<220>
   <223> nucleotide sequence of H3-heavy
<400> 48
   gaggtgcagc tggtggagtc tgggggaggc ttggtccagc ctggagggtc cctgagactc 60
   tcctgtgcag cctctggatt caccttcact gactactaca tgagctgggt ccgccaggct 120
   ccagggaagg ggctggagtg gttgggcttt attagaaaca aagctaacgg ttacaccaca 180
   gaatacagtg cgtctgtgaa aggcagattc accatctcaa gagataattc aaagaactca 240
   ctgtatctgc aaatgaacag cctgcgtgct gaggacacgg ccgtgtatta ctgtgctaga 300
   gataactggt ttgcttactg gggtcaagga accctggtca ccgtctcctc ggctagcacc 360
   aagggcccat cggtcttccc cctggcaccc tcctccaaga gcacctctgg gggcacagcg 420
   gccctgggct gcctggtcaa ggactacttc cccgaaccgg tgacggtgtc gtggaactca 480
   ggcgccctga ccagcggcgt gcacaccttc ccggctgtcc tacagtcctc aggactctac 540
   tccctcagca gcgtggtgac cgtgccctcc agcagcttgg gcacccagac ctacatctgc 600
   aacgtgaatc acaagcccag caacaccaag gtggacaaga aagttgagcc caaatcttgt 660
   gacaaaactc acacatgccc accgtgccca gcacctgaac tcctgggggg accgtcagtc 720
   ttcctcttcc ccccaaaacc caaggacacc ctcatgatct cccggacccc tgaggtcaca 780
   tgcgtggtgg tggacgtgag ccacgaagac cctgaggtca agttcaactg gtacgtggac 840
   ggcgtggagg tgcataatgc caagacaaag ccgcgggagg agcagtacaa cagcacgtac 900
   cgtgtggtca gcgtcctcac cgtcctgcac caggactggc tgaatggcaa ggagtacaag 960
   tgcaaggtct ccaacaaagc cctcccagcc cccatcgaga aaaccatctc caaagccaaa 1020
   gggcagcccc gagaaccaca ggtgtacacc ctgcccccat cccgggagga gatgaccaag 1080
   aaccaggtca gcctgacctg cctggtcaaa ggcttctatc ccagcgacat cgccgtggag 1140
   tgggagagca atgggcagcc ggagaacaac tacaagacca cgcctcccgt gctggactcc 1200
   gacggctcct tcttcctcta cagcaagctc accgtggaca agagcaggtg gcagcagggg 1260
   aacgtcttct catgctccgt gatgcatgag gctctgcaca accactacac gcagaagagc 1320
   ctctccctgt ctccgggtaa atgactcgag 1350
<210> 49
   <211> 1350
   <212> DNA
   <213> Artificial Sequence
<220>
   <223> nucleotide sequence of H4-heavy
<400> 49
   gaggttcagc tggtggagtc tggcggtggc ctggtgcagc cagggggctc actccgtttg      60
   tcctgtgcag cttctggctt caccttcact gattactaca tgagctgggt gcgtcaggcc     120
   ccgggtaagg gcctggaatg gttgggtttt attagaaaca aagctaatgg ttacacaaca     180
   gagtacagtg catctgtgaa gggtcgtttc actataagca gagataattc caaaaacaca     240
   ctgtacctgc agatgaacag cctgcgtgct gaggacactg ccgtctatta ttgtgctaga     300
   gataactggt ttgcttactg gggccaaggg actctggtca ccgtctcctc ggctagcacc     360
   aagggcccat cggtcttccc cctggcaccc tcctccaaga gcacctctgg gggcacagcg     420
   gccctgggct gcctggtcaa ggactacttc cccgaaccgg tgacggtgtc gtggaactca     480
   ggcgccctga ccagcggcgt gcacaccttc ccggctgtcc tacagtcctc aggactctac     540
   tccctcagca gcgtggtgac cgtgccctcc agcagcttgg gcacccagac ctacatctgc     600
   aacgtgaatc acaagcccag caacaccaag gtggacaaga aagttgagcc caaatcttgt     660
   gacaaaactc acacatgccc accgtgccca gcacctgaac tcctgggggg accgtcagtc     720
   ttcctcttcc ccccaaaacc caaggacacc ctcatgatct cccggacccc tgaggtcaca     780
   tgcgtggtgg tggacgtgag ccacgaagac cctgaggtca agttcaactg gtacgtggac     840
   ggcgtggagg tgcataatgc caagacaaag ccgcgggagg agcagtacaa cagcacgtac     900
   cgtgtggtca gcgtcctcac cgtcctgcac caggactggc tgaatggcaa ggagtacaag     960
   tgcaaggtct ccaacaaagc cctcccagcc cccatcgaga aaaccatctc caaagccaaa    1020
   gggcagcccc gagaaccaca ggtgtacacc ctgcccccat cccgggagga gatgaccaag    1080
   aaccaggtca gcctgacctg cctggtcaaa ggcttctatc ccagcgacat cgccgtggag    1140
   tgggagagca atgggcagcc ggagaacaac tacaagacca cgcctcccgt gctggactcc    1200
   gacggctcct tcttcctcta cagcaagctc accgtggaca agagcaggtg gcagcagggg    1260
   aacgtcttct catgctccgt gatgcatgag gctctgcaca accactacac gcagaagagc    1320
   ctctccctgt ctccgggtaa atgactcgag                                     1350
<210> 50
   <211> 669
   <212> DNA
   <213> Artificial Sequence
<220>
   <223> nucleotide sequence of H1-light
<400> 50
   gacatcgtga tgacccagtc tccagactcc ctggctgtgt ctctgggcga gagggccacc 60
   atcaactgca agtccagcca gagtctttta gctagcggca accaaaataa ctacttagct 120
   tggcaccagc agaaaccagg acagcctcct aagatgctca ttatttgggc atctacccgg 180
   gtatccgggg tccctgaccg attcagtggc agcgggtctg ggacagattt cactctcacc 240
   atcagcagcc tgcaggctga agatgtggca gtttattact gtcagcaatc ctatagtgct 300
   cctctcacgt tcggaggcgg taccaaggtg gagatcaaac gtacggtggc tgcaccatct 360
   gtcttcatct tcccgccatc tgatgagcag ttgaaatctg gaactgcctc tgttgtgtgc 420
   ctgctgaata acttctatcc cagagaggcc aaagtacagt ggaaggtgga taacgccctc 480
   caatcgggta actcccagga gagtgtcaca gagcaggaca gcaaggacag cacctacagc 540
   ctcagcagca ccctgacgct gagcaaagca gactacgaga aacacaaagt ctacgcctgc 600
   gaagtcaccc atcagggcct gagctcgccc gtcacaaaga gcttcaacag gggagagtgt 660
   tgactcgag 669
<210> 51
   <211> 669
   <212> DNA
   <213> Artificial Sequence
<220>
   <223> nucleotide sequence of H2-light
<400> 51
   gatattgtga tgacccagac tccactctcc ctgcccgtca cccctggaga gccggcctcc 60
   atctcctgca agtccagtca gagtctttta gctagtggca accaaaataa ctacttggcc 120
   tggcacctgc agaagccagg gcagtctcca cagatgctga tcatttgggc atccactagg 180
   gtatctggag tcccagacag gttcagtggc agtgggtcag gcactgattt cacactgaaa 240
   atcagcaggg tggaggctga ggatgttgga gtttattact gccagcagtc ctacagcgct 300
   ccgctcacgt tcggacaggg taccaagctg gagctcaaac gtacggtggc tgcaccatct 360
   gtcttcatct tcccgccatc tgatgagcag ttgaaatctg gaactgcctc tgttgtgtgc 420
   ctgctgaata acttctatcc cagagaggcc aaagtacagt ggaaggtgga taacgccctc 480
   caatcgggta actcccagga gagtgtcaca gagcaggaca gcaaggacag cacctacagc 540
   ctcagcagca ccctgacgct gagcaaagca gactacgaga aacacaaagt ctacgcctgc 600
   gaagtcaccc atcagggcct gagctcgccc gtcacaaaga gcttcaacag gggagagtgt 660
   tgactcgag 669
<210> 52
   <211> 669
   <212> DNA
   <213> Artificial Sequence
<220>
   <223> nucleotide sequence of H3-light
<400> 52
   gacatcgtga tgacccagtc tccagactcc ctggctgtgt ctctgggcga gagggccacc      60
   atcaactgca agtccagcca gagtctttta gctagcggca accaaaataa ctacttagct     120
   tggtaccagc agaaaccagg acagcctcct aagctgctca ttatttgggc atctacccgg     180
   gtatccgggg tccctgaccg attcagtggc agcgggtctg ggacagattt cactctcacc     240
   atcagcagcc tgcaggctga agatgtggca gtttattact gtcagcaatc ctatagtgct     300
   cctctcacgt tcggaggcgg taccaaggtg gagatcaaac gtacggtggc tgcaccatct     360
   gtcttcatct tcccgccatc tgatgagcag ttgaaatctg gaactgcctc tgttgtgtgc     420
   ctgctgaata acttctatcc cagagaggcc aaagtacagt ggaaggtgga taacgccctc     480
   caatcgggta actcccagga gagtgtcaca gagcaggaca gcaaggacag cacctacagc     540
   ctcagcagca ccctgacgct gagcaaagca gactacgaga aacacaaagt ctacgcctgc     600
   gaagtcaccc atcagggcct gagctcgccc gtcacaaaga gcttcaacag gggagagtgt     660
   tgactcgag                                                             669
<210> 53
   <211> 669
   <212> DNA
   <213> Artificial Sequence
<220>
   <223> nucleotide sequence of H4-light
<400> 53
   gatatccaga tgacccagtc cccgagctcc ctgtccgcct ctgtgggcga tagggtcacc      60
   atcacctgca agtccagtca gagtctttta gctagtggca accaaaataa ctacttggcc     120
   tggcaccaac agaaaccagg aaaagctccg aaaatgctga ttatttgggc atccactagg     180
   gtatctggag tcccttctcg cttctctgga tccgggtctg ggacggattt cactctgacc     240
   atcagcagtc tgcagccgga agacttcgca acttattact gtcagcagtc ctacagcgct     300
   ccgctcacgt tcggacaggg taccaaggtg gagatcaaac gtacggtggc tgcaccatct     360
   gtcttcatct tcccgccatc tgatgagcag ttgaaatctg gaactgcctc tgttgtgtgc     420
   ctgctgaata acttctatcc cagagaggcc aaagtacagt ggaaggtgga taacgccctc     480
   caatcgggta actcccagga gagtgtcaca gagcaggaca gcaaggacag cacctacagc     540
   ctcagcagca ccctgacgct gagcaaagca gactacgaga aacacaaagt ctacgcctgc     600
   gaagtcaccc atcagggcct gagctcgccc gtcacaaaga gcttcaacag gggagagtgt     660
   tgactcgag                                                             669
<210> 54
   <211> 23
   <212> PRT
   <213> Artificial Sequence
<220>
   <223> linker between VH and VL
<400> 54
   Gly Leu Gly Gly Leu Gly Gly Gly Gly Ser Gly Gly Gly Gly Ser Gly
   1               5                   10                  15
   Gly Ser Ser Gly Val Gly Ser
               20
<210> 55
   <211> 1088
   <212> DNA
   <213> Artificial Sequence
<220>
   <223> polynucleotide encoding scFv of huAbF46 antibody
<400> 55
   gctagcgttt tagcagaagt tcaattggtt gaatctggtg gtggtttggt tcaaccaggt      60
   ggttctttga gattgtcttg tgctgcttct ggttttactt tcaccgatta ttacatgtcc     120
   tgggttagac aagctccagg taaaggtttg gaatggttgg gtttcattag aaacaaggct     180
   aacggttaca ctaccgaata ttctgcttct gttaagggta gattcaccat ttctagagac     240
   aactctaaga acaccttgta cttgcaaatg aactccttga gagctgaaga tactgctgtt     300
   tattactgcg ctagagataa ttggtttgct tattggggtc aaggtacttt ggttactgtt     360
   tcttctggcc tcgggggcct cggaggagga ggtagtggcg gaggaggctc cggtggatcc     420
   agcggtgtgg gttccgatat tcaaatgacc caatctccat cttctttgtc tgcttcagtt     480
   ggtgatagag ttaccattac ttgtaagtcc tcccaatctt tgttggcttc tggtaatcag     540
   aacaattact tggcttggca tcaacaaaaa ccaggtaaag ctccaaagat gttgattatt     600
   tgggcttcta ccagagtttc tggtgttcca tctagatttt ctggttctgg ttccggtact     660
   gattttactt tgaccatttc atccttgcaa ccagaagatt tcgctactta ctactgtcaa     720
   caatcttact ctgctccatt gacttttggt caaggtacaa aggtcgaaat caagagagaa     780
   ttcggtaagc ctatccctaa ccctctcctc ggtctcgatt ctacgggtgg tggtggatct     840
   ggtggtggtg gttctggtgg tggtggttct caggaactga caactatatg cgagcaaatc     900
   ccctcaccaa ctttagaatc gacgccgtac tctttgtcaa cgactactat tttggccaac     960
   gggaaggcaa tgcaaggagt ttttgaatat tacaaatcag taacgtttgt cagtaattgc    1020
   ggttctcacc cctcaacaac tagcaaaggc agccccataa acacacagta tgttttttga     1080
   gtttaaac                                                              1088
<210> 56
   <211> 5597
   <212> DNA
   <213> Artificial Sequence
<220>
   <223> expression vector including polynucleotide encoding scFv of huAbF46 antibody
<220>
   <221> misc_difference
   <222> 573..578
   <223> /note="NheI restriction site"
<220>
   <221> misc_difference
   <222> 588..938
   <223> /note="huAbF46 VH"
<220>
   <221> misc_difference
   <222> 939..1007
   <223> /note="linker"
<220>
   <221> misc_difference
   <222> 1008..1349
   <223> /note="huAbF46 VL"
<220>
   <221> misc_difference
   <222> 1350..1355
   <223> /note="EcoRI restriction site"
<220>
   <221> misc_difference
   <222> 1356..1397
   <223> /note="v5 epitope"
<220>
   <221> misc_difference
   <222> 1398..1442
   <223> /note="(G4S)3 linker"
<220>
   <221> misc_difference
   <222> 1443..1649
   <223> /note="Aga2"
<220>
   <221> misc_difference
   <222> 1650..1652
   <223> /note="TGA(stop codon)"
<220>
   <221> misc_difference
   <222> 1653..1660
   <223> /note="PmeI restriction site"
<400> 56
   acggattaga agccgccgag cgggtgacag ccctccgaag gaagactctc ctccgtgcgt 60
   cctcgtcttc accggtcgcg ttcctgaaac gcagatgtgc ctcgcgccgc actgctccga 120
   acaataaaga ttctacaata ctagctttta tggttatgaa gaggaaaaat tggcagtaac 180
   ctggccccac aaaccttcaa atgaacgaat caaattaaca accataggat gataatgcga 240
   ttagtttttt agccttattt ctggggtaat taatcagcga agcgatgatt tttgatctat 300
   taacagatat ataaatgcaa aaactgcata accactttaa ctaatacttt caacattttc 360
   ggtttgtatt acttcttatt caaatgtaat aaaagtatca acaaaaaatt gttaatatac 420
   ctctatactt taacgtcaag gagaaaaaac cccggatcgg actactagca gctgtaatac 480
   gactcactat agggaatatt aagctaattc tacttcatac attttcaatt aagatgcagt 540
   tacttcgctg tttttcaata ttttctgtta ttgctagcgt tttagcagaa gttcaattgg 600
   ttgaatctgg tggtggtttg gttcaaccag gtggttcttt gagattgtct tgtgctgctt 660
   ctggttttac tttcaccgat tattacatgt cctgggttag acaagctcca ggtaaaggtt     720
   tggaatggtt gggtttcatt agaaacaagg ctaacggtta cactaccgaa tattctgctt     780
   ctgttaaggg tagattcacc atttctagag acaactctaa gaacaccttg tacttgcaaa     840
   tgaactcctt gagagctgaa gatactgctg tttattactg cgctagagat aattggtttg     900
   cttattgggg tcaaggtact ttggttactg tttcttctgg cctcgggggc ctcggaggag     960
   gaggtagtgg cggaggaggc tccggtggat ccagcggtgt gggttccgat attcaaatga    1020
   cccaatctcc atcttctttg tctgcttcag ttggtgatag agttaccatt acttgtaagt    1080
   cctcccaatc tttgttggct tctggtaatc agaacaatta cttggcttgg catcaacaaa    1140
   aaccaggtaa agctccaaag atgttgatta tttgggcttc taccagagtt tctggtgttc    1200
   catctagatt ttctggttct ggttccggta ctgattttac tttgaccatt tcatccttgc    1260
   aaccagaaga tttcgctact tactactgtc aacaatctta ctctgctcca ttgacttttg    1320
   gtcaaggtac aaaggtcgaa atcaagagag aattcggtaa gcctatccct aaccctctcc    1380
   tcggtctcga ttctacgggt ggtggtggat ctggtggtgg tggttctggt ggtggtggtt    1440
   ctcaggaact gacaactata tgcgagcaaa tcccctcacc aactttagaa tcgacgccgt    1500
   actctttgtc aacgactact attttggcca acgggaaggc aatgcaagga gtttttgaat    1560
   attacaaatc agtaacgttt gtcagtaatt gcggttctca cccctcaaca actagcaaag    1620
   gcagccccat aaacacacag tatgtttttt gagtttaaac ccgctgatct gataacaaca    1680
   gtgtagatgt aacaaaatcg actttgttcc cactgtactt ttagctcgta caaaatacaa    1740
   tatacttttc atttctccgt aaacaacatg ttttcccatg taatatcctt ttctattttt    1800
   cgttccgtta ccaactttac acatacttta tatagctatt cacttctata cactaaaaaa    1860
   ctaagacaat tttaattttg ctgcctgcca tatttcaatt tgttataaat tcctataatt    1920
   tatcctatta gtagctaaaa aaagatgaat gtgaatcgaa tcctaagaga attgggcaag    1980
   tgcacaaaca atacttaaat aaatactact cagtaataac ctatttctta gcatttttga    2040
   cgaaatttgc tattttgtta gagtctttta caccatttgt ctccacacct ccgcttacat    2100
   caacaccaat aacgccattt aatctaagcg catcaccaac attttctggc gtcagtccac    2160
   cagctaacat aaaatgtaag ctctcggggc tctcttgcct tccaacccag tcagaaatcg    2220
   agttccaatc caaaagttca cctgtcccac ctgcttctga atcaaacaag ggaataaacg    2280
   aatgaggttt ctgtgaagct gcactgagta gtatgttgca gtcttttgga aatacgagtc    2340
   ttttaataac tggcaaaccg aggaactctt ggtattcttg ccacgactca tctccgtgca    2400
   gttggacgat atcaatgccg taatcattga ccagagccaa aacatcctcc ttaggttgat    2460
   tacgaaacac gccaaccaag tatttcggag tgcctgaact atttttatat gcttttacaa    2520
   gacttgaaat tttccttgca ataaccgggt caattgttct ctttctattg ggcacacata    2580
   taatacccag caagtcagca tcggaatcta gagcacattc tgcggcctct gtgctctgca    2640
   agccgcaaac tttcaccaat ggaccagaac tacctgtgaa attaataaca gacatactcc    2700
   aagctgcctt tgtgtgctta atcacgtata ctcacgtgct caatagtcac caatgccctc 2760
   cctcttggcc ctctcctttt cttttttcga ccgaatttct tgaagacgaa agggcctcgt 2820
   gatacgccta tttttatagg ttaatgtcat gataataatg gtttcttagg acggatcgct 2880
   tgcctgtaac ttacacgcgc ctcgtatctt ttaatgatgg aataatttgg gaatttactc 2940
   tgtgtttatt tatttttatg ttttgtattt ggattttaga aagtaaataa agaaggtaga 3000
   agagttacgg aatgaagaaa aaaaaataaa caaaggttta aaaaatttca acaaaaagcg 3060
   tactttacat atatatttat tagacaagaa aagcagatta aatagatata cattcgatta 3120
   acgataagta aaatgtaaaa tcacaggatt ttcgtgtgtg gtcttctaca cagacaagat 3180
   gaaacaattc ggcattaata cctgagagca ggaagagcaa gataaaaggt agtatttgtt 3240
   ggcgatcccc ctagagtctt ttacatcttc ggaaaacaaa aactattttt tctttaattt 3300
   ctttttttac tttctatttt taatttatat atttatatta aaaaatttaa attataatta 3360
   tttttatagc acgtgatgaa aaggacccag gtggcacttt tcggggaaat gtgcgcggaa 3420
   cccctatttg tttatttttc taaatacatt caaatatgta tccgctcatg agacaataac 3480
   cctgataaat gcttcaataa tattgaaaaa ggaagagtat gagtattcaa catttccgtg 3540
   tcgcccttat tccctttttt gcggcatttt gccttcctgt ttttgctcac ccagaaacgc 3600
   tggtgaaagt aaaagatgct gaagatcagt tgggtgcacg agtgggttac atcgaactgg 3660
   atctcaacag cggtaagatc cttgagagtt ttcgccccga agaacgtttt ccaatgatga 3720
   gcacttttaa agttctgcta tgtggcgcgg tattatcccg tgttgacgcc gggcaagagc 3780
   aactcggtcg ccgcatacac tattctcaga atgacttggt tgagtactca ccagtcacag 3840
   aaaagcatct tacggatggc atgacagtaa gagaattatg cagtgctgcc ataaccatga 3900
   gtgataacac tgcggccaac ttacttctga caacgatcgg aggaccgaag gagctaaccg 3960
   cttttttgca caacatgggg gatcatgtaa ctcgccttga tcgttgggaa ccggagctga 4020
   atgaagccat accaaacgac gagcgtgaca ccacgatgcc tgtagcaatg gcaacaacgt 4080
   tgcgcaaact attaactggc gaactactta ctctagcttc ccggcaacaa ttaatagact 4140
   ggatggaggc ggataaagtt gcaggaccac ttctgcgctc ggcccttccg gctggctggt 4200
   ttattgctga taaatctgga gccggtgagc gtgggtctcg cggtatcatt gcagcactgg 4260
   ggccagatgg taagccctcc cgtatcgtag ttatctacac gacgggcagt caggcaacta 4320
   tggatgaacg aaatagacag atcgctgaga taggtgcctc actgattaag cattggtaac 4380
   tgtcagacca agtttactca tatatacttt agattgattt aaaacttcat ttttaattta 4440
   aaaggatcta ggtgaagatc ctttttgata atctcatgac caaaatccct taacgtgagt 4500
   tttcgttcca ctgagcgtca gaccccgtag aaaagatcaa aggatcttct tgagatcctt 4560
   tttttctgcg cgtaatctgc tgcttgcaaa caaaaaaacc accgctacca gcggtggttt 4620
   gtttgccgga tcaagagcta ccaactcttt ttccgaaggt aactggcttc agcagagcgc 4680
   agataccaaa tactgtcctt ctagtgtagc cgtagttagg ccaccacttc aagaactctg 4740
   tagcaccgcc tacatacctc gctctgctaa tcctgttacc agtggctgct gccagtggcg 4800
   ataagtcgtg tcttaccggg ttggactcaa gacgatagtt accggataag gcgcagcggt 4860
   cgggctgaac ggggggttcg tgcacacagc ccagcttgga gcgaacgacc tacaccgaac 4920
   tgagatacct acagcgtgag cattgagaaa gcgccacgct tcccgaaggg agaaaggcgg 4980
   acaggtatcc ggtaagcggc agggtcggaa caggagagcg cacgagggag cttccagggg 5040
   ggaacgcctg gtatctttat agtcctgtcg ggtttcgcca cctctgactt gagcgtcgat 5100
   ttttgtgatg ctcgtcaggg gggccgagcc tatggaaaaa cgccagcaac gcggcctttt 5160
   tacggttcct ggccttttgc tggccttttg ctcacatgtt ctttcctgcg ttatcccctg 5220
   attctgtgga taaccgtatt accgcctttg agtgagctga taccgctcgc cgcagccgaa 5280
   cgaccgagcg cagcgagtca gtgagcgagg aagcggaaga gcgcccaata cgcaaaccgc 5340
   ctctccccgc gcgttggccg attcattaat gcagctggca cgacaggttt cccgactgga 5400
   aagcgggcag tgagcgcaac gcaattaatg tgagttacct cactcattag gcaccccagg 5460
   ctttacactt tatgcttccg gctcctatgt tgtgtggaat tgtgagcgga taacaatttc 5520
   acacaggaaa cagctatgac catgattacg ccaagctcgg aattaaccct cactaaaggg 5580
   aacaaaagct ggctagt 5597
<210> 57
   <211> 13
   <212> PRT
   <213> Artificial Sequence
<220>
   <223> U6-HC7 hinge
<400> 57
   Glu Pro Lys Ser Cys Asp Cys His Cys Pro Pro Cys Pro
   1 5 10
<210> 58
   <211> 435
   <212> DNA
   <213> Artificial Sequence
<220>
   <223> polynucleotide encoding CDR-L3 derived from L3-1 clone
<400> 58
   gaattcacta gtgattaatt cgccgccacc atggattcac aggcccaggt cctcatgttg 60
   ctgctgctat cggtatctgg tacctgtgga gatatccaga tgacccagtc cccgagctcc 120
   ctgtccgcct ctgtgggcga tagggtcacc atcacctgca agtccagtca gagtctttta 180
   gctagtggca accaaaataa ctacttggcc tggcaccaac agaaaccagg aaaagctccg 240
   aaaatgctga ttatttgggc atccactagg gtatctggag tcccttctcg cttctctgga 300
   tccgggtctg ggacggattt cactctgacc atcagcagtc tgcagccgga agacttcgca 360
   acttattact gtcagcagtc ctacagccgc ccgtacacgt tcggacaggg taccaaggtg 420
   gagatcaaac gtacg 435
<210> 59
   <211> 435
   <212> DNA
   <213> Artificial Sequence
<220>
   <223> polynucleotide encoding CDR-L3 derived from L3-2 clone
<400> 59
   gaattcacta gtgattaatt cgccgccacc atggattcac aggcccaggt cctcatgttg 60
   ctgctgctat cggtatctgg tacctgtgga gatatccaga tgacccagtc cccgagctcc 120
   ctgtccgcct ctgtgggcga tagggtcacc atcacctgca agtccagtca gagtctttta 180
   gctagtggca accaaaataa ctacttggcc tggcaccaac agaaaccagg aaaagctccg 240
   aaaatgctga ttatttgggc atccactagg gtatctggag tcccttctcg cttctctgga 300
   tccgggtctg ggacggattt cactctgacc atcagcagtc tgcagccgga agacttcgca 360
   acttattact gtgggcagtc ctacagccgt ccgctcacgt tcggacaggg taccaaggtg 420
   gagatcaaac gtacg 435
<210> 60
   <211> 435
   <212> DNA
   <213> Artificial Sequence
<220>
   <223> polynucleotide encoding CDR-L3 derived from L3-3 clone
<400> 60
   gaattcacta gtgattaatt cgccgccacc atggattcac aggcccaggt cctcatgttg 60
   ctgctgctat cggtatctgg tacctgtgga gatatccaga tgacccagtc cccgagctcc 120
   ctgtccgcct ctgtgggcga tagggtcacc atcacctgca agtccagtca gagtctttta 180
   gctagtggca accaaaataa ctacttggcc tggcaccaac agaaaccagg aaaagctccg 240
   aaaatgctga ttatttgggc atccactagg gtatctggag tcccttctcg cttctctgga 300
   tccgggtctg ggacggattt cactctgacc atcagcagtc tgcagccgga agacttcgca 360
   acttattact gtgcacagtc ctacagccat ccgttctctt tcggacaggg taccaaggtg 420
   gagatcaaac gtacg 435
<210> 61
   <211> 435
   <212> DNA
   <213> Artificial Sequence
<220>
   <223> polynucleotide encoding CDR-L3 derived from L3-5 clone
<400> 61
   gaattcacta gtgattaatt cgccgccacc atggattcac aggcccaggt cctcatgttg 60
   ctgctgctat cggtatctgg tacctgtgga gatatccaga tgacccagtc cccgagctcc 120
   ctgtccgcct ctgtgggcga tagggtcacc atcacctgca agtccagtca gagtctttta 180
   gctagtggca accaaaataa ctacttggcc tggcaccaac agaaaccagg aaaagctccg 240
   aaaatgctga ttatttgggc atccactagg gtatctggag tcccttctcg cttctctgga 300
   tccgggtctg ggacggattt cactctgacc atcagcagtc tgcagccgga agacttcgca 360
   acttattact gtcagcagtc ctacagccgc ccgtttacgt tcggacaggg taccaaggtg 420
   gagatcaaac gtacg 435
<210> 62
   <211> 462
   <212> PRT
   <213> Artificial Sequence
<220>
   <221> SIGNAL
   <222> 1..17
   <223> signal peptide
<220>
   <223> polypeptide consisting of heavy chain of huAbF46-H4-A1, U6-HC7 hinge and constant region of human IgG1
<400> 62
   Met Glu Trp Ser Trp Val Phe Leu Val Thr Leu Leu Asn Gly Ile Gln
   1               5                   10                  15
   Cys Glu Val Gln Leu Val Glu Ser Gly Gly Gly Leu Val Gln Pro Gly
               20                  25                  30
   Gly Ser Leu Arg Leu Ser Cys Ala Ala Ser Gly Phe Thr Phe Thr Asp
           35                  40                  45
   Tyr Tyr Met Ser Trp Val Arg Gln Ala Pro Gly Lys Gly Leu Glu Trp
       50                  55                  60
   Leu Gly Phe Ile Arg Asn Lys Ala Asn Gly Tyr Thr Thr Glu Tyr Ser
   65                  70                  75                  80
   Ala Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Asp Asn Trp Phe Ala Tyr Trp Gly Gln Gly Thr
           115                 120                 125
   Leu Val Thr Val Ser Ser Ala Ser Thr Lys Gly Pro Ser Val Phe Pro
       130                 135                 140
   Leu Ala Pro Ser Ser Lys Ser Thr Ser Gly Gly Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Ser Leu Gly Thr Gln Thr Tyr Ile Cys Asn Val Asn His Lys Pro Ser
       210                 215                 220
   Asn Thr Lys Val Asp Lys Lys Val Glu Pro Lys Ser Cys Asp Cys His
   225                 230                 235                 240
   Cys Pro Pro Cys Pro Ala Pro Glu Leu Leu Gly Gly Pro Ser Val Phe
                   245                 250                 255
   Leu Phe Pro Pro Lys Pro Lys Asp Thr Leu Met Ile Ser Arg Thr Pro
               260                 265                 270
   Glu Val Thr Cys Val Val Val Asp Val Ser His Glu Asp Pro Glu Val
           275                 280                 285
   Lys Phe Asn Trp Tyr Val Asp Gly Val Glu Val His Asn Ala Lys Thr
       290                 295                 300
   Lys Pro Arg Glu Glu Gln Tyr Asn Ser Thr Tyr Arg Val Val Ser Val
   305                 310                 315                 320
   Leu Thr Val Leu His Gln Asp Trp Leu Asn Gly Lys Glu Tyr Lys Cys
                   325                 330                 335
   Lys Val Ser Asn Lys Ala Leu Pro Ala Pro Ile Glu Lys Thr Ile Ser
               340                 345                 350
   Lys Ala Lys Gly Gln Pro Arg Glu Pro Gln Val Tyr Thr Leu Pro Pro
           355                 360                 365
   Ser Arg Glu Glu Met Thr Lys Asn Gln Val Ser Leu Thr Cys Leu Val
       370                 375                 380
   Lys Gly Phe Tyr Pro Ser Asp Ile Ala Val Glu Trp Glu Ser Asn Gly
   385                 390                 395                 400
   Gln Pro Glu Asn Asn Tyr Lys Thr Thr Pro Pro Val Leu Asp Ser Asp
                   405                 410                 415
   Gly Ser Phe Phe Leu Tyr Ser Lys Leu Thr Val Asp Lys Ser Arg Trp
               420                 425                 430
   Gln Gln Gly Asn Val Phe Ser Cys Ser Val Met His Glu Ala Leu His
           435                 440                 445
   Asn His Tyr Thr Gln Lys Ser Leu Ser Leu Ser Pro Gly Lys
       450                 455                 460
<210> 63
   <211> 1410
   <212> DNA
   <213> Artificial Sequence
<220>
   <223> polynucleotide encoding polypeptide consisting of heavy chain of huAbF46-H4-A1, U6-HC7 hinge and constant region of human IgG1
<400> 63
   gaattcgccg ccaccatgga atggagctgg gtttttctcg taacactttt aaatggtatc      60
   cagtgtgagg ttcagctggt ggagtctggc ggtggcctgg tgcagccagg gggctcactc     120
   cgtttgtcct gtgcagcttc tggcttcacc ttcactgatt actacatgag ctgggtgcgt     180
   caggccccgg gtaagggcct ggaatggttg ggttttatta gaaacaaagc taatggttac     240
   acaacagagt acagtgcatc tgtgaagggt cgtttcacta taagcagaga taattccaaa     300
   aacacactgt acctgcagat gaacagcctg cgtgctgagg acactgccgt ctattattgt     360
   gctagagata actggtttgc ttactggggc caagggactc tggtcaccgt ctcctcggct     420
   agcaccaagg gcccatcggt cttccccctg gcaccctcct ccaagagcac ctctgggggc     480
   acagcggccc tgggctgcct ggtcaaggac tacttccccg aaccggtgac ggtgtcgtgg     540
   aactcaggcg ccctgaccag cggcgtgcac accttcccgg ctgtcctaca gtcctcagga     600
   ctctactccc tcagcagcgt ggtgaccgtg ccctccagca gcttgggcac ccagacctac     660
   atctgcaacg tgaatcacaa gcccagcaac accaaggtgg acaagaaagt tgagcccaaa     720
   agctgcgatt gccactgtcc tccatgtcca gcacctgaac tcctgggggg accgtcagtc     780
   ttcctcttcc ccccaaaacc caaggacacc ctcatgatct cccggacccc tgaggtcaca     840
   tgcgtggtgg tggacgtgag ccacgaagac cctgaggtca agttcaactg gtacgtggac     900
   ggcgtggagg tgcataatgc caagacaaag ccgcgggagg agcagtacaa cagcacgtac     960
   cgtgtggtca gcgtcctcac cgtcctgcac caggactggc tgaatggcaa ggagtacaag    1020
   tgcaaggtct ccaacaaagc cctcccagcc cccatcgaga aaaccatctc caaagccaaa    1080
   gggcagcccc gagaaccaca ggtgtacacc ctgcccccat cccgggagga gatgaccaag    1140
   aaccaggtca gcctgacctg cctggtcaaa ggcttctatc ccagcgacat cgccgtggag    1200
   tgggagagca atgggcagcc ggagaacaac tacaagacca cgcctcccgt gctggactcc    1260
   gacggctcct tcttcctcta cagcaagctc accgtggaca agagcaggtg gcagcagggg    1320
   aacgtcttct catgctccgt gatgcatgag gctctgcaca accactacac gcagaagagc    1380
   ctctccctgt ctccgggtaa atgactcgag                                    1410
<210> 64
   <211> 461
   <212> PRT
   <213> Artificial Sequence
<220>
   <221> SIGNAL
   <222> 1..17
   <223> signal peptide
<220>
   <223> polypeptide consisting of heavy chain of huAbF46-H4-A1, human IgG2 hinge and constant region of human IgG1
<400> 64
   Met Glu Trp Ser Trp Val Phe Leu Val Thr Leu Leu Asn Gly Ile Gln
   1               5                   10                  15
   Cys Glu Val Gln Leu Val Glu Ser Gly Gly Gly Leu Val Gln Pro Gly
               20                  25                  30
   Gly Ser Leu Arg Leu Ser Cys Ala Ala Ser Gly Phe Thr Phe Thr Asp
           35                  40                  45
   Tyr Tyr Met Ser Trp Val Arg Gln Ala Pro Gly Lys Gly Leu Glu Trp
       50                  55                  60
   Leu Gly Phe Ile Arg Asn Lys Ala Asn Gly Tyr Thr Thr Glu Tyr Ser
   65                  70                  75                  80
   Ala Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Asp Asn Trp Phe Ala Tyr Trp Gly Gln Gly Thr
           115                 120                 125
   Leu Val Thr Val Ser Ser Ala Ser Thr Lys Gly Pro Ser Val Phe Pro
       130                 135                 140
   Leu Ala Pro Ser Ser Lys Ser Thr Ser Gly Gly Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Ser Leu Gly Thr Gln Thr Tyr Ile Cys Asn Val Asn His Lys Pro Ser
       210                 215                 220
   Asn Thr Lys Val Asp Lys Lys Val Glu Arg Lys Cys Cys Val Glu Cys
   225                 230                 235                 240
   Pro Pro Cys Pro Ala Pro Glu Leu Leu Gly Gly Pro Ser Val Phe Leu
                   245                 250                 255
   Phe Pro Pro Lys Pro Lys Asp Thr Leu Met Ile Ser Arg Thr Pro Glu
               260                 265                 270
   Val Thr Cys Val Val Val Asp Val Ser His Glu Asp Pro Glu Val Lys
           275                 280                 285
   Phe Asn Trp Tyr Val Asp Gly Val Glu Val His Asn Ala Lys Thr Lys
       290                 295                 300
   Pro Arg Glu Glu Gln Tyr Asn Ser Thr Tyr Arg Val Val Ser Val Leu
   305                 310                 315                 320
   Thr Val Leu His Gln Asp Trp Leu Asn Gly Lys Glu Tyr Lys Cys Lys
                   325                 330                 335
   Val Ser Asn Lys Ala Leu Pro Ala Pro Ile Glu Lys Thr Ile Ser Lys
               340                 345                 350
   Ala Lys Gly Gln Pro Arg Glu Pro Gln Val Tyr Thr Leu Pro Pro Ser
           355                 360                 365
   Arg Glu Glu Met Thr Lys Asn Gln Val Ser Leu Thr Cys Leu Val Lys
       370                 375                 380
   Gly Phe Tyr Pro Ser Asp Ile Ala Val Glu Trp Glu Ser Asn Gly Gln
   385                 390                 395                 400
   Pro Glu Asn Asn Tyr Lys Thr Thr Pro Pro Val Leu Asp Ser Asp Gly
                   405                 410                 415
   Ser Phe Phe Leu Tyr Ser Lys Leu Thr Val Asp Lys Ser Arg Trp Gln
               420                 425                 430
   Gln Gly Asn Val Phe Ser Cys Ser Val Met His Glu Ala Leu His Asn
           435                 440                 445
   His Tyr Thr Gln Lys Ser Leu Ser Leu Ser Pro Gly Lys
       450                 455                 460
<210> 65
   <211> 1407
   <212> DNA
   <213> Artificial Sequence
<220>
   <223> polynucleotide encoding polypeptide consisting of heavy chain of huAbF46-H4-A1, human IgG2 hinge and constant region of human IgG1
<400> 65
   gaattcgccg ccaccatgga atggagctgg gtttttctcg taacactttt aaatggtatc      60
   cagtgtgagg ttcagctggt ggagtctggc ggtggcctgg tgcagccagg gggctcactc     120
   cgtttgtcct gtgcagcttc tggcttcacc ttcactgatt actacatgag ctgggtgcgt     180
   caggccccgg gtaagggcct ggaatggttg ggttttatta gaaacaaagc taatggttac     240
   acaacagagt acagtgcatc tgtgaagggt cgtttcacta taagcagaga taattccaaa     300
   aacacactgt acctgcagat gaacagcctg cgtgctgagg acactgccgt ctattattgt     360
   gctagagata actggtttgc ttactggggc caagggactc tggtcaccgt ctcctcggct     420
   agcaccaagg gcccatcggt cttccccctg gcaccctcct ccaagagcac ctctgggggc     480
   acagcggccc tgggctgcct ggtcaaggac tacttccccg aaccggtgac ggtgtcgtgg     540
   aactcaggcg ccctgaccag cggcgtgcac accttcccgg ctgtcctaca gtcctcagga     600
   ctctactccc tcagcagcgt ggtgaccgtg ccctccagca gcttgggcac ccagacctac     660
   atctgcaacg tgaatcacaa gcccagcaac accaaggtgg acaagaaagt tgagaggaag     720
   tgctgtgtgg agtgcccccc ctgcccagca cctgaactcc tggggggacc gtcagtcttc     780
   ctcttccccc caaaacccaa ggacaccctc atgatctccc ggacccctga ggtcacatgc     840
   gtggtggtgg acgtgagcca cgaagaccct gaggtcaagt tcaactggta cgtggacggc     900
   gtggaggtgc ataatgccaa gacaaagccg cgggaggagc agtacaacag cacgtaccgt     960
   gtggtcagcg tcctcaccgt cctgcaccag gactggctga atggcaagga gtacaagtgc    1020
   aaggtctcca acaaagccct cccagccccc atcgagaaaa ccatctccaa agccaaaggg    1080
   cagccccgag aaccacaggt gtacaccctg cccccatccc gggaggagat gaccaagaac    1140
   caggtcagcc tgacctgcct ggtcaaaggc ttctatccca gcgacatcgc cgtggagtgg    1200
   gagagcaatg ggcagccgga gaacaactac aagaccacgc ctcccgtgct ggactccgac    1260
   ggctccttct tcctctacag caagctcacc gtggacaaga gcaggtggca gcaggggaac    1320
   gtcttctcat gctccgtgat gcatgaggct ctgcacaacc actacacgca gaagagcctc    1380
   tccctgtctc cgggtaaatg actcgag                                        1407
<210> 66
   <211> 460
   <212> PRT
   <213> Artificial Sequence
<220>
   <221> SIGNAL
   <222> 1..17
   <223> signal peptide
<220>
   <223> polypeptide consisting of heavy chain of huAbF46-H4-A1, human IgG2 hinge and constant region of human IgG2
<400> 66
   Met Glu Trp Ser Trp Val Phe Leu Val Thr Leu Leu Asn Gly Ile Gln
   1               5                   10                  15
   Cys Glu Val Gln Leu Val Glu Ser Gly Gly Gly Leu Val Gln Pro Gly
               20                  25                  30
   Gly Ser Leu Arg Leu Ser Cys Ala Ala Ser Gly Phe Thr Phe Thr Asp
           35                  40                  45
   Tyr Tyr Met Ser Trp Val Arg Gln Ala Pro Gly Lys Gly Leu Glu Trp
       50                  55                  60
   Leu Gly Phe Ile Arg Asn Lys Ala Asn Gly Tyr Thr Thr Glu Tyr Ser
   65                  70                  75                  80
   Ala Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Asp Asn Trp Phe Ala Tyr Trp Gly Gln Gly Thr
           115                 120                 125
   Leu Val Thr Val Ser Ser Ala Ser Thr Lys Gly Pro Ser Val Phe Pro
       130                 135                 140
   Leu Ala Pro Cys Ser Arg Ser Thr Ser Glu Ser Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Asn Phe Gly Thr Gln Thr Tyr Thr Cys Asn Val Asp His Lys Pro Ser
       210                 215                 220
   Asn Thr Lys Val Asp Lys Thr Val Glu Arg Lys Cys Cys Val Glu Cys
   225                 230                 235                 240
   Pro Pro Cys Pro Ala Pro Pro Val Ala Gly Pro Ser Val Phe Leu Phe
                   245                 250                 255
   Pro Pro Lys Pro Lys Asp Thr Leu Met Ile Ser Arg Thr Pro Glu Val
               260                 265                 270
   Thr Cys Val Val Val Asp Val Ser His Glu Asp Pro Glu Val Gln Phe
           275                 280                 285
   Asn Trp Tyr Val Asp Gly Val Glu Val His Asn Ala Lys Thr Lys Pro
       290                 295                 300
   Arg Glu Glu Gln Phe Asn Ser Thr Phe Arg Val Val Ser Val Leu Thr
   305                 310                 315                 320
   Val Val His Gln Asp Trp Leu Asn Gly Lys Glu Tyr Lys Cys Lys Val
                   325                 330                 335
   Ser Asn Lys Gly Leu Pro Ala Pro Ile Glu Lys Thr Ile Ser Lys Thr
               340                 345                 350
   Lys Gly Gln Pro Arg Glu Pro Gln Val Tyr Thr Leu Pro Pro Ser Arg
           355                 360                 365
   Glu Glu Met Thr Lys Asn Gln Val Ser Leu Thr Cys Leu Val Lys Gly
       370                 375                 380
   Phe Tyr Pro Ser Asp Ile Ala Val Glu Trp Glu Ser Asn Gly Gln Pro
   385                 390                 395                 400
   Glu Asn Asn Tyr Lys Thr Thr Pro Pro Met Leu Asp Ser Asp Gly Ser
                   405                 410                 415
   Phe Phe Leu Tyr Ser Lys Leu Thr Val Asp Lys Ser Arg Trp Gln Gln
               420                 425                 430
   Gly Asn Val Phe Ser Cys Ser Val Met His Glu Ala Leu His Asn His
           435                 440                 445
   Tyr Thr Gln Lys Ser Leu Ser Leu Ser Pro Gly Lys
       450                 455                 460
<210> 67
   <211> 1404
   <212> DNA
   <213> Artificial Sequence
<220>
   <223> polynucleotide encoding polypeptide consisting of heavy chain of huAbF46-H4-A1, human IgG2 hinge and constant region of human IgG2
<400> 67
   gaattcgccg ccaccatgga atggagctgg gtttttctcg taacactttt aaatggtatc      60
   cagtgtgagg ttcagctggt ggagtctggc ggtggcctgg tgcagccagg gggctcactc     120
   cgtttgtcct gtgcagcttc tggcttcacc ttcactgatt actacatgag ctgggtgcgt     180
   caggccccgg gtaagggcct ggaatggttg ggttttatta gaaacaaagc taatggttac     240
   acaacagagt acagtgcatc tgtgaagggt cgtttcacta taagcagaga taattccaaa     300
   aacacactgt acctgcagat gaacagcctg cgtgctgagg acactgccgt ctattattgt     360
   gctagagata actggtttgc ttactggggc caagggactc tggtcaccgt ctcctcggct     420
   agcaccaagg gcccatcggt cttccccctg gcgccctgct ccaggagcac ctccgagagc     480
   acagcggccc tgggctgcct ggtcaaggac tacttccccg aaccggtgac ggtgtcgtgg     540
   aactcaggcg ctctgaccag cggcgtgcac accttcccag ctgtcctaca gtcctcagga     600
   ctctactccc tcagcagcgt ggtgaccgtg ccctccagca acttcggcac ccagacctac     660
   acctgcaacg tagatcacaa gcccagcaac accaaggtgg acaagacagt tgagcgcaaa     720
   tgttgtgtcg agtgcccacc gtgcccagca ccacctgtgg caggaccgtc agtcttcctc     780
   ttccccccaa aacccaagga caccctcatg atctcccgga cccctgaggt cacgtgcgtg     840
   gtggtggacg tgagccacga agaccccgag gtccagttca actggtacgt ggacggcgtg     900
   gaggtgcata atgccaagac aaagccacgg gaggagcagt tcaacagcac gttccgtgtg     960
   gtcagcgtcc tcaccgttgt gcaccaggac tggctgaacg gcaaggagta caagtgcaag    1020
   gtctccaaca aaggcctccc agcccccatc gagaaaacca tctccaaaac caaagggcag    1080
   ccccgagaac cacaggtgta caccctgccc ccatcccggg aggagatgac caagaaccag    1140
   gtcagcctga cctgcctggt caaaggcttc taccccagcg acatcgccgt ggagtgggag    1200
   agcaatgggc agccggagaa caactacaag accacgcctc ccatgctgga ctccgacggc    1260
   tccttcttcc tctacagcaa gctcaccgtg gacaagagca ggtggcagca ggggaacgtc    1320
   ttctcatgct ccgtgatgca tgaggctctg cacaaccact acacgcagaa gagcctctcc    1380
   ctgtctccgg gtaaatgact cgag                                           1404
<210> 68
   <211> 240
   <212> PRT
   <213> Artificial Sequence
<220>
   <221> SIGNAL
   <222> 1..20
   <223> signal peptide
<220>
   <223> polypeptide consisting of light chain of huAbF46-H4-A1(H36Y) and human kappa constant region
<400> 68
<210> 69
   <211> 758
   <212> DNA
   <213> Artificial Sequence
<220>
   <223> polynucleotide encoding polypeptide consisting of light chain of hUAbF46-H4-A1(H36Y) and human kappa constant region
<400> 69
   aattcactag tgattaattc gccgccacca tggattcaca ggcccaggtc ctcatgttgc      60
   tgctgctatc ggtatctggt acctgtggag atatccagat gacccagtcc ccgagctccc     120
   tgtccgcctc tgtgggcgat agggtcacca tcacctgcaa gtccagtcag agtcttttag     180
   ctagtggcaa ccaaaataac tacttggcct ggtaccaaca gaaaccagga aaagctccga      240
   aaatgctgat tatttgggca tccactaggg tatctggagt cccttctcgc ttctctggat      300
   ccgggtctgg gacggatttc actctgacca tcagcagtct gcagccggaa gacttcgcaa      360
   cttattactg tcagcagtcc tacagccgcc cgtacacgtt cggacagggt accaaggtgg      420
   agatcaaacg tacggtggct gcaccatctg tcttcatctt cccgccatct gatgagcagt      480
   tgaaatctgg aactgcctct gttgtgtgcc tgctgaataa cttctatccc agagaggcca      540
   aagtacagtg gaaggtggat aacgccctcc aatcgggtaa ctcccaggag agtgtcacag      600
   agcaggacag caaggacagc acctacagcc tcagcagcac cctgacgctg agcaaagcag      660
   actacgagaa acacaaagtc tacgcctgcg aagtcaccca tcagggcctg agctcgcccg      720
   tcacaaagag cttcaacagg ggagagtgtt gactcgag                              758
<210> 70
   <211> 240
   <212> PRT
   <213> Artificial Sequence
<220>
   <221> SIGNAL
   <222> 1..20
   <223> signal peptide
<220>
   <223> polypeptide consisting of light chain of huAbF46-H4-A1 and human kappa constant region
<400> 70
   Met Asp Ser Gln Ala Gln Val Leu Met Leu Leu Leu Leu Ser Val Ser
   1               5                   10                  15
   Gly Thr Cys Gly Asp Ile Gln Met Thr Gln Ser Pro Ser Ser Leu Ser
               20                  25                  30
   Ala Ser Val Gly Asp Arg Val Thr Ile Thr Cys Lys Ser Ser Gln Ser
           35                  40                  45
   Leu Leu Ala Ser Gly Asn Gln Asn Asn Tyr Leu Ala Trp His Gln Gln
       50                  55                  60
   Lys Pro Gly Lys Ala Pro Lys Met Leu Ile Ile Trp Ala Ser Thr Arg
   65                  70                  75                  80
   Val Ser Gly Val Pro Ser Arg Phe Ser Gly Ser Gly Ser Gly Thr Asp
                   85                  90                  95
   Phe Thr Leu Thr Ile Ser Ser Leu Gln Pro Glu Asp Phe Ala Thr Tyr
               100                 105                 110
   Tyr Cys Gln Gln Ser Tyr Ser Arg Pro Tyr Thr Phe Gly Gln Gly Thr
           115                 120                 125
   Lys Val Glu Ile Lys Arg Thr Val Ala Ala Pro Ser Val Phe Ile Phe
       130                 135                 140
   Pro Pro Ser Asp Glu Gln Leu Lys Ser Gly Thr Ala Ser Val Val Cys
   145                 150                 155                 160
   Leu Leu Asn Asn Phe Tyr Pro Arg Glu Ala Lys Val Gln Trp Lys Val
                   165                 170                 175
   Asp Asn Ala Leu Gln Ser Gly Asn Ser Gln Glu Ser Val Thr Glu Gln
               180                 185                 190
   Asp Ser Lys Asp Ser Thr Tyr Ser Leu Ser Ser Thr Leu Thr Leu Ser
           195                 200                 205
   Lys Ala Asp Tyr Glu Lys His Lys Val Tyr Ala Cys Glu Val Thr His
       210                 215                 220
   Gln Gly Leu Ser Ser Pro Val Thr Lys Ser Phe Asn Arg Gly Glu Cys
   225                 230                 235                 240
<210> 71
   <211> 17
   <212> PRT
   <213> Artificial Sequence
<220>
   <223> CDR-L1 of antibody L3-11Y
<400> 71
   Lys Ser Ser Gln Ser Leu Leu Ala Trp Gly Asn Gln Asn Asn Tyr Leu
   1               5                   10                  15
   Ala
<210> 72
   <211> 114
   <212> PRT
   <213> Artificial Sequence
<220>
   <223> amino acid sequence of light chain variable region of antibody L3-11Y
<400> 72
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Trp
               20                  25                  30
   Gly Asn Gln Asn Asn Tyr Leu Ala Trp Tyr Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
   65                  70                  75                  80
   Ile Ser Ser Leu Gln Pro Glu Asp Phe Ala Thr Tyr Tyr Cys Gln Gln
                   85                  90                  95
   Ser Tyr Ser Arg Pro Tyr Thr Phe Gly Gln Gly Thr Lys Val Glu Ile
               100                 105                 110
   Lys Arg
<210> 73
   <211> 220
   <212> PRT
   <213> Artificial Sequence
<220>
   <223> amino acid sequence of light chain of antibody L3-11Y
<400> 73
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Trp
               20                  25                  30
   Gly Asn Gln Asn Asn Tyr Leu Ala Trp Tyr Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
   65                  70                  75                  80
   Ile Ser Ser Leu Gln Pro Glu Asp Phe Ala Thr Tyr Tyr Cys Gln Gln
                   85                  90                  95
   Ser Tyr Ser Arg Pro Tyr Thr Phe Gly Gln Gly Thr Lys Val Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220